# EUROPEAN PATENT SPECIFICATION

(11) **EP 0 813 423 B1**
(45) Date of publication and mention of the grant of the patent: **03.07.2002**
(21) Application number: 96903630.0
(22) Date of filing: 23.01.1996
(51) Int. Cl.: A61K 39/395, A61K 31/36

(54) **COMPOSITION TO INHIBIT OSTEOLYSIS AND METASTASIS**
ZUSAMMENSETZUNG ZUR VERHINDERUNG VON OSTEOLYSE UND METASTASEN
PROCEDE VISANT A INHIBER L'OSTEOLYSE ET LES METASTASES

(30) Priority: 23.01.1995 US 376359; 09.02.1995 US 386361; 06.06.1995 US 481088
(43) Date of publication of application: 29.12.1997
(73) Proprietor: Xenotech Incorporated, Fremont, CA 94555 (US)
(72) Inventor: MUNDY, Gregory, R., San Antonio, TX 78230 (US); YONEDA, Toshiyuki, San Antonio, TX 78230 (US); GUISE, Theresa, A., San Antonio, TX 78230 (US)
(74) Representative: Goldin, Douglas Michael
(86) International application number: US9600895
(87) International publication number: WO9622790

(56) References cited:
- EP-A- 0 092 004
- EP-A- 0 148 075
- EP-A- 0 267 611
- EP-A- 0 355 067
- EP-A- 0 509 553
- EP-A- 0 626 389
- WO-A-88/00596
- WO-A-89/07453
- WO-A-94/21266
- WO-A-95/01997
- WO-A-95/14780
- US-A- 5 124 147
- US-A- 5 183 657
- US-A- 5 334 380
- H. FLEISCH : "Bisphosphonates. Pharmacology and Use in the Treatment of Tumour-Induced Hypercalcaemic and Metastatic Bone Disease" DRUGS, vol. 42, no. 6, 1991, pages 914-944, XP000676675
- PATENT ABSTRACTS OF JAPAN vol. 16, no. 575 (C-1011), 15 December 1992 & JP 04 228089 A (KANEGAFUCHI CHEM IND CO LTD), 18 August 1992,

## Description

### Technical Field

The invention relates to the prevention and treatment of cancer metastasis to bone and bone cancer growth and to associated osteolysis, some of the most problematic aspects of cancers of many origins. More specifically, the invention concerns use of antibodies to parathyroid hormone-related protein (PTH-rp) and other forms of anti-PTH-rp alone or in combination with a bisphosphonate to alleviate these conditions.

### Background of the Invention

It has long been understood that morbidity and mortality caused by cancer, especially in view of the fact that surgical techniques are readily available, are due to more-or-less systemic sequelae of the original multiplication of the cancer cells. Metastasis of an original tumor to additional locations, the destruction of target locations as a direct or indirect result of this metastasis, cachexia, hypercalcemia, and other symptomologies characterize the course of the malignancy. The mechanisms whereby these sequelae occur are believed to involve a variety of cytokines, growth factors, and cell adhesion molecules, among other factors. It is entirely unknown, however, how cancers dissolve bone in bone metastases since at present, the only cells known to be able to dissolve bone are osteoclasts. It is not known how metastasized cancer dissolves bone in order to accommodate the growth of the cancer.

One of many factors which is known to play a role in at least some of these processes is parathyroid hormone-related protein (PTH-rp). Due to its similarity to PTH, it has been recognized as a mediator of humoral hypercalcemia of malignancy, HHM (WO-A-88/00596). In this role, PTH-rp secreted by the tumor is circulated through the blood and is associated with hypercalcemia, an index of bone resorption.

PTH-rp has been shown to be the cause of systemic bone resorption and calcium reabsorption from urine at the tubule. Both of these result in a systemic elevation of blood calcium levels. This factor has been described in connection with HHM in U.S. Patent 5,116,952. This patent also describes preparation of anti-PTH-rp antibodies using peptide subunits of the partially sequenced protein. JP-A-04/228089 also describes the preparation of antibodies against PTH-P. This protein has also been called adenylate cyclase stimulating factor (ACSF) in U.S. Patent 5,312,810 and humoral hypercalcemic factor (hHCF) in U.S. Patent 5,114,843. The association of PTH-rp with HHM is also described by Yates, A.J.P. et al., J Clin Invest (1988) 81:932-938. Japanese application A4-228089, published 18 August 1992 describes recombinant production of anti-PTH-rp antibodies, including murine/human chimeras. This application also discloses animal studies demonstrating that either murine or chimeric monoclonal antibodies directed to PTH-rp ameliorate the effects of HHM in a model system where HHM is caused by either systemically infusing PTH-rp into mice peritoneally or by implanting PTH-rp-producing prostate cancer cell lines in nude mice.

PTH-rp has been purified from human lung cancer, breast cancer and renal cell carcinoma. Although PTH-rp was originally found associated with tumor cells, it is also present widely in normal tissue. The gene encoding PTH-ro was cloned and expressed by Suva, L.J. et al. Science (1987) 237:893-895. PTH-rp has been shown to bind to PTH receptors (Ahou-Samra, A. et al. Proc Natl Acad Sci USA (1992) 89:2732-2736) and has activities similar to PTH (Horiuchi, N. et al. Science (1987) 23:1566-1568). It has been shown to stimulate adenylate cyclase in renal and bone systems, increase tubular reabsorption of calcium, decrease renal phosphate uptake and stimulate 1α-hydroxylase. Thus, it is logical that where PTH-rp is circulating, inhibitors of the PTH-like action of PTH-rp would interfere with these biological events, including calcium reabsorption from urine to blood at the kidney tubules and systemic bone resorption. However, PTH/PTH-rp receptors are not found on osteoclasts. Either such receptors have been resistant to characterization or to the extent that PTH-rp may be found to affect osteoclasts, the effect may be exerted by some unknown mechanism, for example by effecting the secretion of unknown mediators from osteoblasts (which are known to have PTH/PTH-rp receptors). There is no available evidence to connect PTH-rp to bone resorption mediated by osteoclasts.

PTH-rp also has properties that are not shared by PTH, including regulation of placental calcium transport as summarized in a review by Mallette, L.E. Endocrine Rev (1991) 12:110-117. Of particular relevance with respect to the invention herein is that PTH-rp has also been associated with the establishment of bone metastasis in breast cancer (Powell, G.J. Cancer Res (1991) 51:3059-3061; Southby, J. et al. Cancer Res (1990) 50:7710-7716). The data provided in these disclosures show only the association of PTH-rp with bone metastasis of breast cancer, but no causal relationships are implied. PTH-rp has been considered as a possible autocrine factor for a limited number of tumors (Burton, P.B.J. et al. Biochem Biophys Res Com (1990) 167:1134 1138; Li, X. et al. Cancer Res (1993) 53:2980-2986). Thus, the precise role of PTH-rp in mediating the effects of a primary cancer is not well understood. Not only are there additional factors which also participate in the progress of this condition, the production of PTH-rp is believed affected by additional factors such as prolactin, glucocorticoids, epidermal growth factor, TGF-α, TGF-β, estrogen, "stretch," and even extracellular calcium concentration. The presence of additional factors is particularly important and adds to the uncertainty of the mechanism. Factors such as IL-1, IL-6, IL-11, M-CSF and GM-CSF in particular have been considered important in bone resorption. Synthetic compounds such as bisphosphates can also have an effect on bone resorption (Flersch et al., Drugs (1991) 42:919-944). In a report by Sasaki, A. et al. J Bone Min Res (1994) 9 Supp 1:S 294, B 257, it is demonstrated that administration of recombinant human IL-1α into calvaria. of nude mice attracted metastasis of cancer cells that had been inoculated into the left ventricle, while other bones of the same animal which did not receive IL-1α did not show cancer metastasis. Gilles, *J. et al. ibid.* S 337, B 433, suggest that TL-6 markedly potentiates the effects of bone resorbing factors on osteoclastic bone resorption and that IL-6 production in bone is important both in cytokine-mediated bone resorption and in those disease states where.cytokine-mediaced bone resorption is potentially involved, such as estrogen-related bone loss, myeloma and Paget's Disease. It has also been shown that transgenic mice that are modified to comprise an IL-6 gene knockout show reduced bone loss in models for osteoporosis. It has been confirmed that osteoclasts have receptors for IL-6 and IL-11. Osteoclasts treated with M-CSF and GM-CSF show accelerated maturation in culture. Transgenic mice that have genetic deficiencies in the M-CSF gene also exhibit a condition mimicking osteoporosis and this condition can be treated by injection of M-CSF. Thus, a multiplicity of factors are known which appear to play a major role in local bone resorption and/or osteoclast activation.

Antibodies have been raised against some of the factors which are thought to play a role in bone resorption including TNF-α (EPA-0626,389, EP-A-0355,067, US Patent 5,183,657, US Patent 5,124,147 and US Patent 5,344,380), IL-1 (WO-A-95/01997 and EP-A-0267,611), lymphotoxin (EP-A-509,553), prostaglandins (EP-A-148,075) and vitamin D3 derivatives (EP-A-092,004).

Sato, K. *et al* J. Bone Min Res (1993) 8:849-860 describe results obtained in a murine model of HHM wherein the affected mice were administered a monoclonal murine antibody obtained from immortalized spleen cells of mice injected with peptide representing amino acid positions 1-34 of PTH-rp. Passive immunization resulted in decreases in serum calcium concentration in these hypercalcemic nude mice that had been transplanted with human PTH-rp-producing tumors. These tumors were implanted in nonbone tissue to provide a model for HHM. The authors suggest that if a human counterpart to this antibody could be obtained, it might be used in treatment where malignancy-associated hypercalcemia is due to PTH-rp. (Sato, K. *et al.* use the term "malignancy associated hypercalcemia" in this paper; it is synonymous with HHM). In this model, elevated levels of PTH-rp are maintained in the blood which are, evidently, mitigated by the administration of anti-PTH-rp antibodies. Similar studies were conducted by Kukreja, S.C. J. clin Invest (1988) 82:1798-1802 and showed that polyclonal anti-PTH-rp antibodies suppress hypercalcemia in a human tumor model of HHM. This is the first disclosure of any therapeutic effect of anti-PTH-rp on HHM. The relevance of this model is verified by the finding of Tashjian, A.H. *et al* J Exp Med (1964) 119:467 that 15% of 147 hypercalcemic breast cancer patients exhibited no bone metastases.

Hypercalcemia may also be caused by osteolysis of bone through the ediation of osteoclasts. Munday, G.R. J Clin Invest (1988) 82:1-6 describes increased osteoclastic bone resorption in areas surrounding breast cancer metastases. In addition, the breast cancer cells have been shown to resorb bone directly in vitro (Eilon, G. & Mundy G. R., Nature (1978) 276:726-728.

Kohno, N. et al. Surgery Today, Japan J Surg (1993) 4:215-220 reported studies performed on immobilized sections of surgically removed breast cancers using an anti-PTH-rp antibody also prepared by immunizing mice with the first 34 amino acids of PTH-rp. The antibody bound to 57% of the tumors; it bound to 83% of the tumors derived from patients who developed skeletal metastases but only 38% in those who either developed lung metastases or no metastases at all. These authors conclude that their results suggest that PTH-rp-positive tumors have an affinity to bone. However, the observations related only to the staining of cancer tissues by anti-PTH-rp and are not quantitative. Thus, the results do not show a causal relationship between the presence of PTH-rp and any acceleration of bone metastasis or growth enhancers. An equally plausible explanation is that PTH-rp is an effect rather than a cause of metastasis, cancer growth and/or osteolysis. The present inventors have used their animal model to develop an effective treatment for the localized effects of metastasized cancer cells on bone and thereby developed the present invention.

### Summary of the Invention

The invention includes several aspects each of which provides for the treatment of the localized effects of metastasized cancer cells on bone. Treatment is carried out by administering a therapeutically effective amount of a pharmaceutically acceptable formulation comprised of a carrier with one or more antibodies alone or in combination with one or more bisphosphonates.

Specific localized effects can be obtained, i.e., cancer metastases to the bone can be prevented, growth of cancer cells present in bone can be inhibited, and osteolytic effects can be prevented and treated. Further, the secondary effects of the metastasis, growth, and osteolysis associated with localized phenomena in bone can be mitigated.

The carrier is preferably a pharmaceutically acceptable injectable carrier. The antibodies are immunoreactive with and bind to a compound selected from the group consisting of PTHrp, TGFα, IL-1α, IL-1β, IL-6, Lymphotoxin, TNF, PGE, and 1,25 dihydroxy vitamin D₃. Antibodies for PTHrp are preferred and are preferably administered by injection in an injectable formulation comprised of anti-PTHrp alone or in combination with one or more bisphosphonates.

Antibodies for PTHrp are preferably administered by I.V. injection in an injectable formulation. The formulation can be administered along with separate administration of a bisphosphonate. Alternatively, antibodies for PTHrp can be formulated together with and administered with a bisphosphonate. Useful bisphosphonates include etidronate pamidronate, risedronate, pyrophosphate, clodronate, tiludronate, alendronate, BM 21.0955, YM-175, CGP42446 and pharmaceutically acceptable salts, hydrates and partial hydrates thereof. By combining antibodies of the type described above with bisphosphonates it is possible to obtain synergistic results in terms of the treatments of the localized effects of metastasized cancer cells on bone due to different mechanisms of action of the antibodies as compared to the bisphosphonates.

Bisphosphonates such as pamidronate disodium are referred to as amino-substituted bisphosphonate compound which compounds rapidly chemisorb to calcium on the surface of hydroxyapatite crystals, preferentially in areas of high bone turnover, thereby reducing the solubility of the mineralized matrix and rendering it more resistant to osteoclastic resorption. The attachment of osteoclastic precursors to the mineralized matrix is impaired when previously bound pamidronate is released in high local concentrations from the matrix surface during the early phase of resorption. Accordingly, transformation of precursors into mature, functioning osteoclasts and dissolution of bone matrix are then inhibited.

In another aspect, the invention is directed to a method to inhibit osteolysis effected by metastasis of cancer cells to bone and/or a method to prevent cancer metastasis to bone and/or a method to inhibit growth of metastasized or bone-originated cancer cells in or on bone which method comprises administering to a subject in need of such treatment an amount of a specific antibody (e.g., anti-PTH-rp) alone or in combination with a bisphosphonate in an amount sufficient to have a therapeutically useful effect.

Another effect of the method of the invention in attacking the primary targets described above is the amelioration of secondary symptomologies. These include pain, neural compression which may result in paraplegia or paralysis, hypercalcemia, risk of pathologic fractures, cachexia and decreased survival prognosis.

In still other aspects, the invention is directed to compositions useful in the methods of the invention and to methods to identify and treat subjects who would benefit from the methods of invention treatment, as well as methods to obtain antibodies useful in the methods of the invention.

Based on the above it can be seen that a primary object of the present invention is to provide a pharmaceutically active formulation useful in a method of localized treatment which allows for the localized inhibition of osteolysis effected by metastasis of cancer cells to bone. The method is carried out by the administration of a therapeutically effective amount of an injectable formulation comprising an antibody immunoreactive with a compound selected from the group consisting of PTHrp, TGFα, IL-1α, IL-1β, IL-6, Lymphotoxin, TNF, PGE; 1,25 dihydroxy vitamin D₃ and an antigenic fragment thereof, anti-PTH-rp alone or in combination with a bisphosphonate.

Another object of the invention is to provide formulations which are preferably in the form of injectable formulations for the prevention of cancer metastases to bone comprising a pharmaceutically acceptable, injectable carrier with a protein antibody therein alone or in combination with a bisphosphonate.

Another object of the invention is to provide formulations which are preferably in the form of injectable formulations for the inhibition of growth of cancer cells present in bone comprising a pharmaceutically acceptable, injectable carrier with a protein antibody therein alone or in combination with a bisphosphonate.

Another object of the invention is to provide formulations which are preferably in the form of injectable formulations for the prevention of osteolysis comprising a pharmaceutically acceptable, injectable carrier with a protein antibody therein alone or in combination with a bisphosphonate.

A feature of the invention is that the protein antibodies and bisphosphonates are relatively non-toxic especially as compared to conventional anti-cancer compounds.

An advantage of the invention is that the localized effect of inhibiting osteolysis effected by metastasis is unexpectedly additive i.e., the effect of the protein antibodies is additive to the effect of the bisphosphonates thereby providing a synergistic effect due to different mechanisms of action.

These and other objects, advantages and features of the present invention will become apparent to those persons skilled in the art upon reading the details of these formulations and treatment protocols as more fully set forth below reference being made to the accompanying figures forming a part hereof.

### Brief Description of the Drawing

Figure 1 is a graph that shows the effect of monoclonal antibodies specific for PTH-rp on serum calcium levels in tumor-bearing mice;
Figure 2 is a graph showing the effect of monoclonal antibodies specific for PTH-rp on calcium release in bone resorption stimulated by PTH-rp, PTH, IL-1 or 1,25-dihydroxy vitamin D₃ in an organ culture model;
Figure 3 is a graph showing the effect of administering anti-PTH-rp antibodies on the total area shown to be associated with bone lesions by x-ray diagnosis in tumor-bearing mice;
Figure 4 is a graph showing the results of the experimental protocol described in Example 3;
Figure 5 is a graph showing the effect of PTP-rp antibody on stimulated mouse bone marrow;
Figure 6 shows the general structure of immunoglobulins; and
Figure 7 is a graph showing the concentration of PTHrp in control mice and an animal as per Exampple 5.

### Description of the Preferred Embodiments

Before the present method of inhibiting osteolysis effected by metastasis of cancer cells and formulations used in such are described, it is to be understood that this invention is not limited to the particular antibodies, bisphosphonates, or formulations and methodologies described as such may, of course, vary. It is also to be understood that the terminology used herein is for the purpose of describing particular embodiments only, and is not intended to limit the scope of the present invention which will be limited only by the appended claims.

It must be noted that as used in this specification and the appended claims, the singular forms "a", "an", and "the" include plural referents unless the context clearly dictates otherwise. Thus, for example, reference to "a bisphosphonate" includes mixtures of bisphosphonates and multiple copies of molecules of a particular bisphosphonate, reference to "an antibody" includes a plurality of antibodies and reference to "the method" includes one or more methods which would become apparent to those skilled in the art upon reading this disclosure and which would be useful in carrying out the invention.

Unless defined otherwise all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### Definitions

As used herein, the term "anti-PTH-rp" refers to antibodies immunoreactive with PTH-rp and antibody - analogs, substitutes and fragments as are known in the art. It is preferred that the anti-PTH-rp also be immunospecific -- i.e., not substantially cross-reactive with related materials such as PTH. However, anti-PTH-rp is effective in the method of the invention as long as it is immunoreactive with PTH-rp *per se.* All of the invention methods and compositions employ substances immunoreactive or immunospecific for and therefore specifically and selectively bind to a compound selected from the group consisting of PTHrp, TGFα, IL-1α, IL-1β, IL-6, Lymphotoxin, TNF, PGE, and 1,25 dihydroxy vitamin D₃ or an antigenic fragment thereof. Substances that are immunoreactive with PTH-rp are generally designated "anti-PTH-rp" and compounds immunoreactive with TGFα are generally designated "anti-TGFα", etc. Compounds which are immunoreactive and immunospecific for PTH-rp are preferred.

By "purified antibody" is meant one which is sufficiently free of other proteins, carbohydrates, and lipids with which it is naturally associated. Such an antibody "preferentially binds" to a compound selected from the group consisting of PTHrp, TGFα, IL-1α, IL-1β, IL-6, Lymphotoxin, TNF, PGE; and 1,25 dihydroxy vitamin D₃ (or an antigenic fragment thereof), i.e., does not substantially recognize and bind to other antigenically-unrelated molecules. A purified antibody of the invention is preferably immunoreactive with and immunospecific for a compound selected from the group consisting of PTHrp, TGFα, IL-1α, IL-1β, IL-6, Lymphotoxin, TNF, PGE; and 1,25 dihydroxy vitamin D₃ (or an antigenic fragment thereof).

By "antigenic fragment" of a compound selected from the group consisting of PTHrp, TGFα, IL-1α, IL-1β, IL-6, Lymphotoxin, TNF, PGE; and 1,25 dihydroxy vitamin D₃ is meant a portion of such a compound which is capable of binding an antibody generated by immunization of a mammal with such a compound or a fragment thereof. Preferably, the antibodies which specifically bind an epitope of the isolated antigenic fragment will also bind the same epitope in the context of the native protein from which the fragment was derived.

By "antibody" is meant an immunoglobulin protein which is capable of binding an antigen. Antibody as used herein is meant to include antibody fragments (e.g. F(ab')₂, Fab', Fab) capable of binding the antigen or antigenic fragment of interest.

The term "humanized antibody" is used herein to describe complete antibody molecules (i.e., composed of two complete light chains and two complete heavy chains), as well antibodies consisting only of antibody fragments (e.g., Fab, Fab', F(ab')2, and Fv), wherein the CDRs are derived from a non-human source and the remaining portion of the Ig molecule or fragment thereof is derived from a human antibody preferable produced from a nucleic acid sequence encoding a human antibody.

The term "human body" is used herein to describe an antibody of which all portions of the antibody molecule are derived from a nucleic acid sequence encoding a human antibody. Such human antibodies are most desirable for use in antibody therapies, as such antibodies would elicit little or no immune response in the human patient.

The term "chimeric antibody" is used herein to describe an antibody molecule as well as antibody fragments as described above in the definition of the term "humanized antibody." The term "chimeric antibody" encompasses humanized antibodies. Chimeric antibodies have at least one portion of a heavy or light chain amino acid sequence derived from a first mammalian species and another portion of the heavy or light chain amino acid sequence derived from a second, different mammalian species. Preferably, the variable region is derived from a non-human mammalian species and the constant region is derived from a human species. Specifically, the chimeric antibody is preferably produced from a nucleotide sequence from a non-human mammal encoding a variable region and a nucleotide sequence from a human encoding a constant region of an antibody.

By "binds specifically" is meant high avidity and/or high affinity binding of an antibody to a specific polypeptide. Antibody binding to its epitope on this specific polypeptide is stronger than binding of the same antibody to any other epitope, particularly those which may be present in molecules in association with, or in the same sample, as the specific polypeptide of interest. Antibodies which bind specifically to a polypeptide of interest may be capable of binding other polypeptides at a weak, yet detectable, level (e.g., 10% or less of the binding shown to the polypeptide of interest). Such weak binding, or background binding, is readily discernible from the specific antibody binding to the compound or polypeptide of interest, e.g. by use of appropriate controls.

By "detectably labeled antibody", "detectably labeled anti-PTHrp" or "detectably labeled anti-PTHrp fragment" is meant an antibody (or antibody fragment which retains binding specificity), having an attached detectable label. The detectable label is normally attached by chemical conjugation, but where the label is a polypeptide, it could alternatively be attached by genetic engineering techniques. Methods for production of detectably labeled proteins are well known in the art. Detectable labels may be selected from a variety of such labels known in the art, but normally are radioisotopes, fluorophores, enzymes (e.g., horseradish peroxidase), or other moieties or compounds which either emit a detectable signal (e.g., radioactivity, fluorescence, color) or emit a detectable signal after exposure of the label to its substrate. Various detectable label/substrate pairs (e.g., horseradish peroxidase/diaminobenzidine, avidin/streptavidin, luciferase/luciferin)), methods for labelling antibodies, and methods for using labeled antibodies to detect an antigen (such as a compound selected from the group consisting of PTHrp, TGFα, IL-1α, IL-1β, IL-6, Lymphotoxin, TNF, PGE; and 1,25 dihydroxy vitamin D₃) are well known in the art (see, for example, Harlow and Lane, eds. (*Antibodies: A Laboratory Manual* (1988) Cold Spring Harbor Laboratory Press, Cold Spring Harbor, NY)).

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease in a mammal, particularly a human, and includes:
(a) preventing the disease from occurring in a subject which may be predisposed to the disease but has not yet been diagnosed as having it;
(b) inhibiting the disease, i.e., arresting its development; or
(c) relieving the disease, i.e., causing regression of the disease. The invention is directed toward treating patients with cancer and is particularly directed toward treating cancer cell metastasis to the bone locally i.e., preventing, inhibiting or relieving cancer cell metastasis at one or more particular sites. In terms of treating symptoms the invention is directed toward treating neural compression; hypercalcemia; reduced life span; pathologic fractures; and cachexia. The treatment is particularly directed toward treating metastasis of cancer cells selected from the group consisting of squamous cell carcinoma cells, adenocarcinoma cells, melanoma cells, osteosarcoma cells, neuroblastoma cells, or hematologic cancer cells. More specifically, "treatment" is intended to mean providing a therapeutically detectable and beneficial effect on a patient suffering from cancer metastasis to bone and cancer cell growth in bone as well as osteolysis and symptomatic sequelae thereof.

Still more specifically "treatment" shall mean preventing, alleviating, and/or inhibiting (1) osteolysis mediated by bone-localized production of a compound selected from the group consisting of PTHrp, TGFα, IL-1α, IL-1β, IL-6, Lymphotoxin, TNF, PGE; 1,25 dihydroxy vitamin D₃ and an antigenic fragment thereof; and particularly bone-localized production of parathyroid hormone-related protein (PTHrp). Specific treatments are directed at preventing or inhibiting abnormal osteolysis mediated by bone-localized production of PTHrp. Treatment includes preventing or inhibiting bone resorption caused by and/or related to cancer cell metastasis to bone.

The terms "synergistic", "synergistic effect" and like are used herein to describe improved treatment effects obtained by combining one or more antibodies with one or more bisphosphonates. Although a synergistic effect in some field is meant an effect which is more than additive (e.g., 1+1=3) in the field of cancer therapy an additive (1+1=2) or less than additive (1+1=1.6) effect may be synergistic. For example, if each of two drugs were to slow the growth of cancer cells by 50% if given individually it would not be expected that the two drugs would be combined to completely stop tumor growth. In many instances, due to unacceptable side effects, the two drugs can not be administered together. In other instances the drugs counteract each other and slow tumor growth by less than 50% when administered together. Thus, a synergistic effect is said to be obtained if the two drugs slow tumor growth by more than either drug alone (preferably decrease growth by 50% or more compared to either drug alone) while not causing an unacceptable increase in adverse side effects e.g., increased toxicity.

The term "therapeutic index" refers to the therapeutic index of a compound defined as LD₅₀/ED₅₀. The LD₅₀ (lethal dose, 50%) is defined as the dose of a compound which kills 50% of the animals tested, and the ED₅₀ is defined as the effective dose of the compound for 50% of the individuals. Compounds with a therapeutic index near unity (i.e., LD₅₀/ED₅₀ is approximately equal to 1) achieve their desired effect at doses very close to the toxic level and as such have a narrow therapeutic window, i.e., a narrow dose range over which they may be administered.

### Antibody Production

Polyclonal antibodies and/or monoclonal antibodies that specifically bind to a compound selected from the group consisting of PTHrp, TGFα, IL-1α, IL-1β, IL-6, Lymphotoxin, TNF, PGE; and 1,25 dihydroxy vitamin D₃ or antigenic fragments thereof can be generated according to methods well known and routine in the art (see, for example, Harlow and Lane, 1988, Antibodies: A Laboratory Manual, Cold Spring Harbor Laboratory Press, Cold Spring Harbor, NY; Schrier et al., 1980, Hybridoma Technigues, Cold Spring Harbor Laboratory Press, Cold Spring Harbor, NY). More specifically, to generate antibodies for use with the present invention one can isolate PTHrp by Superose-12 gel filtration and combine the isolated compound with Freund's complete adjuvant and inject into New Zealand albino rabbits. At 21 day intervals, provide the animals with a booster injection of protein plus Freund's incomplete adjuvant. After the third boost, obtain serum samples and detect antibodies using a conventional dot-blot assay. Determine antibody titers by serial dilutions onto nitrocellulose. Detect antibody binding using the Problot (Promega) system.

Examples of art-known analogs, fragments and substitutes for antibodies include fragments such as Fab, Fab', and F(ab')2 fragments as well as single-chain forms designated Fᵥ fragments obtained recombinantly by fusing the genes encoding the light- and heavy-chain variable regions. Antibodies can be monoclonal or polyclonal and can be those produced in response to administration of an antigen without modification, or, since recombinant techniques have become available, modifications to these antibodies are also possible. For example, also included within anti-PTH-rp are chimeric antibodies which contain regions of amino acid sequences derived from different species, humanized and human antibodies, primatized antibodies as well as modified forms of antibodies generated by one species that have been altered so as to resemble those of another species without altering the antigen specificity. A particularly preferred embodiment of the anti-PTH-rp of the invention includes human antibodies produced in response to administration of PTH-rp to transgenic mice whose immune systems have been modified so that human antibodies can be generated.

Anti-PTH-rp can be prepared in a variety of ways. In one approach, a suitable immunogen, such as PTH-rp or a subunit thereof is administered to a vertebrate capable of an immune response to the immunogen. Particularly preferred subunits of PTH-rp include those in the N-terminal region, in particular positions 1-34. The PTH-rp or subunit used as immunogen should include epitopes characteristic of the particular species PTH-rp for which antibodies are desired. Suitable polyclonal antisera from the immunized animals could be used; however, it is preferred to obtain monoclonal preparations that are reproducible and readily prepared. Such monoclonal preparations can be obtained by immortalizing the antibody-producing cells of the immunized animal and screening for the appropriate immunoreactivity. Immunospecificity can also be ascertained by screening out those hybridomas that secrete antibodies that are crossreactive with closely related materials such as PTH.

The resulting antibodies can be used per se or fragments of these antibodies can be prepared using standard proteolytic techniques to provide the Fab, Fab', and F(ab')₂ fragments. Alternatively, the genes encoding the antibodies may be isolated, for example from the hybridoma, and manipulated to alter the characteristics of the antibody. Using the isolated genes, Fᵥ units can be constructed which contain the light- and heavy-chain variable regions covalently linked in a single peptide unit.

### Chimeric Antibodies

Chimeric antibodies are antibodies having one portion of a heavy or light chain with an amino acid sequence (variable region) that corresponds to a first mammalian species with the remaining segment of the amino acid chain (constant region) corresponding to that of another species. Typically, a chimeric antibody includes the variable region of both heavy and light chains from a first mammalian species (generally non-human) and constant regions corresponding to the sequences of another mammalian species--generally human.

It is desirable to produce chimeric antibodies because the variable regions (which specifically binds to any desired antigen) can be conveniently derived from inexpensive sources such as an inoculated rabbit and reproduced using readily available hybridomas. Those variable regions can be combined with a constant region derived from a human cell line. The resulting chimeric antibody has the advantage of having a variable region which can be readily and inexpensively prepared with any desired specificity using any desired source while keeping a constant region from a human source which is less likely to illicit an immune response from a human subject if the antibodies are administered to a human.

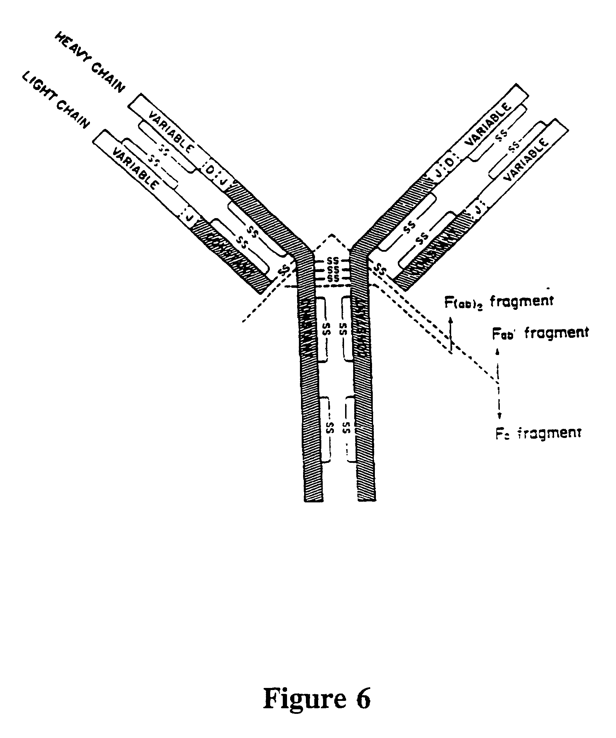

Figure 6 is a representation of the general structure of immunoglobulins. The structural unit includes two identical light polypeptide chains of molecular weight of approximately 23,000 daltons and two identical heavy chains of molecular weight in the range of 53,000-70,000. The four chains are joined by disulfide bonds in a "Y" configuration wherein the light chains bracket the heavy chains starting at the mouth of the "Y" and continuing through the divergent region. The "branch portion" is designated the Fab region. Heavy chains are classified as gamma, mu, alpha, delta, or epsilon with some subclasses. The nature of the heavy chain determines the "class" of the antibody as being IgG, IgM, IgA, IgB, or IgE.

The light chains of the immunoglobulin are classified as either kappa or lambda. Each heavy chain may be prepared with a kappa or lambda light chain. The light and heavy chains are covalently bound to each other, and the "tail" portion of the two heavy chains are bonded to each other by covalent disulfide linkages when the immunoglobulins are generated either by hybridomas or by B cells. Referring to Figure 6, a chimeric antibody is an antibody wherein the white area labelled as "variable" is from a first animal source and the gray area labelled "constant" is from a second, different animal source which is preferably a human.

Chimeric antibodies of the present invention can be understood by reference to the general structure of antibodies as described above and shown in Figure 6 and the general structure and characteristics of chimeric antibodies as described above. Chimeric antibodies can be made by a number of different routes. Initially, chimeric antibodies were produced by chemical synthesis methodology. Methods from chemically synthesizing the chimeric antibodies are disclosed within Bobrzecka, et al. (Bobrzecka, K., Konieczny, L., Laidler, P., and Rybarska, J., 1980. *Immunology Letters.* 2:151-155), and Konieczny, et al. (Konieczny, L., Bobrzecka, K., Laidler, P., and Rybarska, J., 1981. *Haematologia.* 14:95-99), (collectively, the "Rybarska papers"). These papers disclosed the general concept of making chimeric antibodies which is desirable in that the variable portion can be conveniently and inexpensively produced within an animal such as a rabbit and bound to a constant portion which is extracted from a human source. The resulting chimeric is less likely to generate an immune reaction when administered to a human.

The basic technology for making chimeric proteins which would be applicable to chimeric antibodies was known at least as early as 1987 (see U.S. Patent 4,704,362, issued November 3, 1987 to Itakura, et al). A specific example of applying the Itakura, et al technology to producing chimeric antibodies is described within U.S. Patent 4,816,567, issued March 28, 1989 to Cabilly, et al.

DNA sequences including both the variable and constant regions of antibodies from humans as well as many animal sources have been published. It is now relatively routine to isolate genetic material encoding particular antibodies. Using this technology one can isolate the genetic material which encodes the variable region of a rabbit generated antibody immunoreactive and immunospecific for PTH-rp. That DNA sequence can be ligated to a DNA sequence which encodes the constant region of a human antibody. The construct is inserted within an appropriate vector which is then inserted within an appropriate host cell. The host cell may be human and may be cultured or inserted directly into an individual. In either case the transfected cell will generate chimeric antibodies which bind to PTH-rp. In the same manner other chimeric antibodies can be generated wherein the variable portion binds to any of PTH-rp, TGFα, IL-1α, I-L-1β, I-L-6, Lymphotoxin, TNF, PGE, 1,25 Dihydroxy Vitamin D₃ and antigenic fragment thereof. When the resulting chimeric antibody is administered to a human the antibody will not generate an immune response and will be useful in the treatment of cancer metastasis to bone and cancer cell growth as well as osteolysis and the symptomatic sequela thereof.

### Humanized Antibodies

Both non-human antibodies and human/non-human chimeric antibodies elicit an immune response (i.e., are antigenic) when administered to humans. The "humanized antibody" was developed to avoid the antigenicity of antibodies administered to humans, yet retain the antigen specificity of a non-human antibody.

A humanized antibody is a genetically engineered immunoglobulin (Ig) molecule composed of: 1) all or a portion of the complementarity-determining regions (CDRs) of a non-human antibody (e.g., mouse, rat, or rabbit), which confers a desired antigen specificity upon the antibody, 2) framework regions (FRs) derived from a human antibody variable region, and 3) a constant region derived from a human antibody. Thus, only the CDRs are derived from a non-human source; all other portions of the antibody are derived from a human antibody. Such humanized antibody technology allows the use of animals to obtain an antibody of a desired antigen specificity, then uses genetic engineering technology to produce an antibody having the antigen specificity of a non-human antibody without the antigenicity of the non-human antibody. As used herein, "humanized antibody" includes complete antibody molecules (i.e., composed of 2 complete light chains and 2 complete heavy chains), as well as antibody fragments (e.g, Fab, Fab', F(ab')2, and Fv), wherein the CDRs are derived from a non-human source and the remaining portion of the Ig molecule or fragment thereof is derived from a human antibody.

Humanized antibodies can be prepared according to methods known in the art (Jones et al., 1986 *Nature* 321:522; Riechmann et al., 1988 *Nature* 332:323; Verhoeyen et al., 1988 *Science* 239:1534; Presta et al., 1992 *Curr Opin Struc Biol* 2:593; U.S. Patent No. 5,225,539; and PCT Published Application No. WO 91/09968). While the CDRs of the humanized antibody ultimately contact the antigen and determine antigen specificity, the binding activity of the CDRs is regulated by the framework residues (FRs) surrounding the CDRs. Various technical approaches for modifying FRs to alter target antigen binding by the CDRs are described in, for example, Jolliffe et al., 1993 Intern Rev. *Imnunol* 10:241; Junghans et al., 1990 *Cancer Res* 50:1495; Riechmann et al., 1988 *Nature* 332:323; Sim et al., 1993 *J Immunol* 151:2296; Chothia et al., 1987 *J Mol Biol* 196:901; Carter et al., 1992 *Proc Natl Acad Sci USA* 89:4285; and Presta et al., 1993 *J Iminunol* 151:2623. Methods for isolating and manipulating desired nucleic acid sequences (such as those encoding the desired non-human and human antibody portions), and methods for expressing recombinant antibodies *in vitro* are well known in the art (see, for example, Sambrook, et al.,1989, Molecular Cloning: A Laboratory Manual, 2nd Ed., Cold Spring Harbor Laboratory Press, Cold Spring Harbor, NY; Cabilly et al. U.S. Patent No. 4,816,567; and Morrison et al., 1984 *Proc Natl Acad Sci USA* 81:6851).

### Human Antibodies

As used herein, "human antibody" means an antibody of which all portions of the antibody molecule are derived from a nucleic acid sequence encoding a human antibody. Such human antibodies are most desirable for use in antibody therapies, as such antibodies would elicit little or no immune response in the human patient.

Human antibodies can be prepared using a variety of methods well known in the art. For example, human hybridomas expressing human antibodies can be prepared by fusing a human B cell expressing an antibody having a desired antigen specificity with a human myeloma or chimeric (i.e., human/non-human) heteromyeloma. Methods for production of human hybridoma cell lines are well known in the art (see, for example, Kozbor et al., 1984 *J Immunol* 147:86; Bordeur et al., 1987, In: Monoclonal Antibody Production Techniques and Applications, Marcel Dekker, Inc., New York, NY, p. 51-63).

A preferred method for human antibody production uses transgenic animals expressing human Ig genes. Mice are generally the host animal of choice. Such human antibody-producing transgenic animals can be produced using a. combination of techniques to first inactivate the host animal's endogenous Ig genes and then introduce into the host animal's germline DNA the desired human Ig genes (e.g.., human genes encoding an antibody heavy chain and an antibody light chain that, when combined, provide a human antibody of a desired specificity). The transgenic animal is then immunized with the antigen which the human antibody encoded by the transgenes specifically binds to elicit an immune response in the animal, thereby promoting development and maturation of B cells expressing the desired human antibody. Hybridoma cell lines are then prepared from the transgenic host animal using methods well known in the art (see, for example, Kohler et al., 1975, *Nature,* 256:495; Green et al., 1994, *Nature Genetics* 7:13; Kucherlapati et al., PCT Published Application No. WO 94/02602; Nishi, June 1995 *Nikkei Science:* 40; and Cappechi, May 1994, *Nikkei Science* :52).

Still another method for producing human antibodies, and/or fragments thereof, uses phage display techniques, such as those described by, for example, McCafferty et al., 1990 *Nature* 348:552; Johnson et al., 1993 *Curr Opin Struc Biol* 3:564; Clackson et al., 1991 *Nature* 352:624; Marks et al., 1991 *J Mol Biol* 222:581; Griffith et al., 1993 *EMBO J* 12:725; Marks et al., 1992 *Bio-Technol* 10:779; Waterhouse et al., 1993 *Nucl Acid Res* 21:2265; and PCT Published Application No. WO 93/06213.

### Bisphosphonates

Bisphosphonate compounds used in connection with the present invention have the following general structure formula 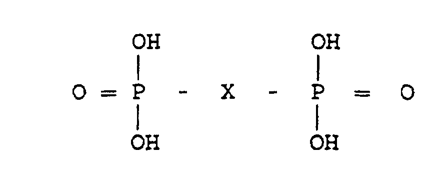 wherein X is a linking moiety allowing for the interconnection of the phosphonate groups. Useful bisphosphonates include pharmaceutically acceptable salts, hydrates as well as multiple and partial hydrates thereof e.g., dihydrates and hemihydrates.

Preferred bisphosphonates are those wherein X is selected from the group consisting of -O-, and 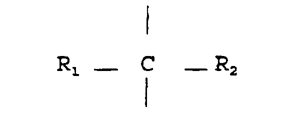 wherein R¹, R² are each independently selected from the group consisting of H, OH, Cl, NH₂, S, thiophenyl wherein the phenyl ring may be substituted with a substituent selected from the group consisting of Cl, OH and HN₂, alkyl containing 1 to 12 carbon atoms, substituted alkyl containing 1 to 12 carbons wherein the substituent is selected from the group consisting of O, S, NH₂, N, and N-alkyl containing 1 to 12 carbon atoms, a cyclic compound containing 1 to 12 carbon atoms any of which carbon atoms are optionally substituted with a substituent from the group consisting of Cl, OH and NH₂ and a heterocyclic compound containing 1 to 12 carbon atoms any of which carbon atoms are optionally substituted with a substituent selected from the group consisting of Cl, OH, NH₂ wherein the hetoatom is selected from the group consisting of O, N, S.

Some more preferred bisphosphonates include those wherein X is selected from the group consisting of -O-, 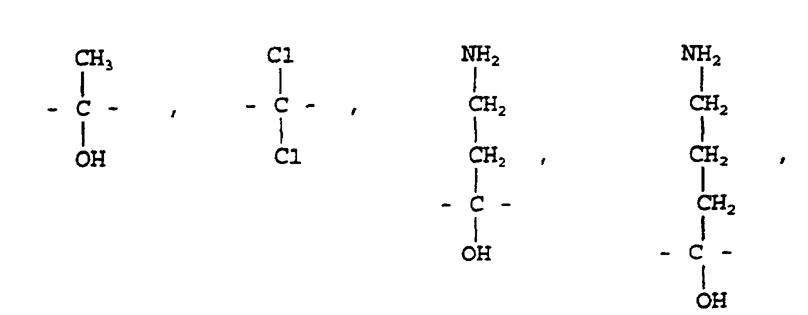 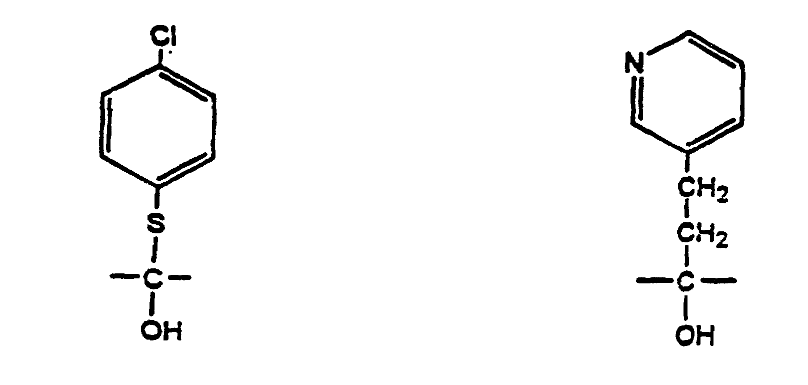 and

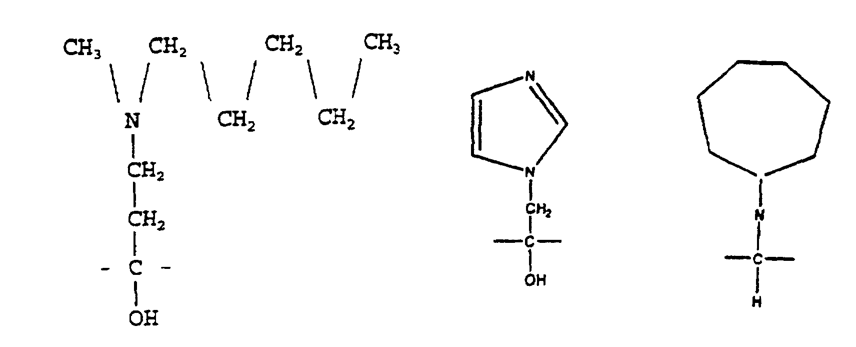

One particularly preferred bisphosphonate is 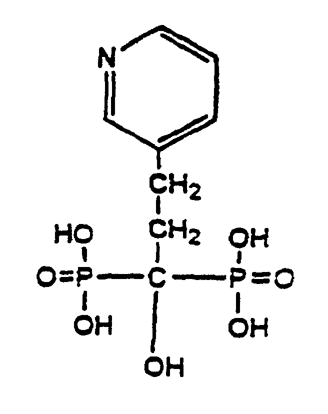 which is included in an injectable pharmaceutical formulation along with a therapeutically effective amount of an antibody which is immunoreactive and immunospecific for a compound selected from the group consisting of PTHrp, TGFα, IL-1α, IL-1β, IL-6, Lymphotoxin, TNF, PGE; 1,25 dihydroxy vitamin D₃ and an antigenic fragment thereof and a pharmaceutically acceptable excipient. Useful bisphosphonates include etidronate pamidronate, risedronate, pyrophosphate, clodronate, tiludronate, alendronate, BM 21.0955, YM-175, CGP42446 and pharmaceutically acceptable salts, hydrates and partial hydrates thereof.

### Formulations - General

The appropriate antibodies or other forms of anti-PTH-rp are formulated for administration in a manner customary for administration of such materials. Typical formulations are those provided in Remington's Pharmaceutical Sciences, latest edition, Mack Publishing Company, Easton, PA. Preferably, the anti-PTH-rp is administered by injection, including intramuscular, intravenous (IV), subcutaneous or peritoneal injection, most preferably IV and local injections. However, other modes of administration may also be used provided means are available to permit the anti-PTH-rp to enter the systemic circulation, such as transmucosal or transdermal formulations, which can be applied as suppositories, skin patches, or intranasally. In addition, local administration such as by cerebrospinal injection or local injection into the bone metastasis sites or fracture sites may also be used. Any suitable formulation which effects the transfer of the anti-PTH-rp to the bloodstream or locally to the bone may properly be used.

For injection, suitable formulations generally comprise aqueous solutions or suspensions using physiological saline, Hank's Solution, or other buffers optionally including stabilizing agents or other minor components. Liposomal preparations and other forms of microemulsions can also be used. The anti-PTH-rp may also be supplied in lyophilized form and reconstituted for administration. Transmucosal and transdermal formulations generally include agents which facilitate transition of the mucosal or dermal barrier, such as bile salts, fusidic acid and its analogs, various detergents, and the like. Oral administration would also be possible provided suitable enteric coatings can be formulated to permit the anti-PTH-rp to survive the digestive tract.

The nature of the formulation will depend to some extent on the nature of the anti-PTH-rp chosen and a suitable formulation is prepared using known techniques and principles of formulation well known to those in the art. The percentage of anti-PTH-rp contained in a particular pharmaceutical composition will also depend on the nature of the formulation; the percentage of anti-PTH-rp will typically vary over a wide range from about 1 by weight to about 85∼ by weight.

### Dosages

The appropriate dosage level will also vary depending on a number of factors including the nature of the subject to be treated, the particular nature of the condition to be treated and its severity, the nature of the antibody used as active ingredient, the mode of administration, the formulation, and the judgment of the practitioner. When antibodies are administered by themselves such as anti-PTHrp in an injectable formulation the dosages will be in the range of 50 µg/kg-50 mg/kg, preferably 1 mg/kg-10 mg/kg at a single dosage. Repeated administration may be required according to protocols to be determined considering the variables set forth above. Typically, daily administration over a period of 3-10 days may be required or administration by intravenous means may be continuous. For chronic conditions, administration may be continued for longer periods as necessary.

As described herein, the subjects who would benefit from administration of any of the antibodies (e.g. anti-PTH-rp) are those who show metastasis of cancer cells to bone or the growth of cancer cells in bone or osteolysis. These patients may or may not have elevated levels of PTH-rp circulating in the blood, unlike those subjects whose hypercalcemia is mediated by the humoral system. As set forth in Japanese Patent Application No. H4-228089, laid open 18 August 1992, hypercalcemia associated with malignant tumors can be caused either by the accelerated bone resorption by invasion of tumor cells into the bone (local osteolytic hypercalcemia (LOH)) or by the systemic effect of PTH-rp (humoral hypercalcemia of malignancy (HHM)). According to this disclosure, the major examples of hypercalcemia caused by LOH are those resulting from bone metastases of multiple myeloma, breast cancer and the like and examples of hypercalcemia resulting from HHM is the condition associated with squamous cell carcinoma and renal/urological cancers. However, Japanese Patent Application No. H4-228089 does not disclose the relevance of PTH-rp to local osteolytic hypercalcemia.

Bisphosphonate compounds are generally administered in an amount in the range of about 30-60 mg bi-weekly to a normal sized adult. Alternatively, bisphosphonates may be administered in an amount of about 60-90 mg on a monthly basis wherein the bisphosphonate is administered over a period of 1-24 hrs. In connection with the present invention the bisphosphonate will be combined with an antibody such as anti-PTH-rp and administered in smaller amounts on a daily basis. For example, bisphosphonates of the type described herein may be administered on a daily basis and in an amount of about 5-25 mg to an adult male of approximately 70 kg. The amount administered can be decreased or increased depending upon the size, age, or condition of the individual being treated. As with any drugs, it is preferable to begin the treatment with a relatively small amount of active ingredient and gradually increase the dosage over time as needed while determining the effects of the drug in the treatment of the patient.

### Formulations - Specific

Formulations of the present invention can be prepared by combining formulations of antibodies with the formulations of bisphosphonates. The two formulations can be administered simultaneously while by being maintained in separate containers. However, it is possible to combine the antibody and bisphosphonate compounds together within a single formulation and administer that formulation to a patient for purposes of treatment. Examples of formulations which can be used in connection with the present invention include the following:

| Formulation No. 1 | |
|---|---|
| Bisphosphonate | 5-25 mg |
| Antibody | 70-700 mg |
| Excipient | 5-25 ml |

| Formulation No. 2 | |
|---|---|
| Bisphosphonate | 5-25 mg |
| Anti-PTHrp | 70-700 mg |
| Excipient | 5-25 ml |

| Formulation No. 3 | |
|---|---|
| Bisphosphonate | 5 mg |
| Anti-TGFα | 70 mg |
| Excipient | 5 ml |

| Formulation No. 4 | |
|---|---|
| Bisphosphonate | 25 mg |
| Anti-IL-1α | 700 mg |
| Excipient | 25 ml |

| Formulation No. 5 | |
|---|---|
| Bisphosphonate | 5 mg |
| Anti-IL-1β | 700 mg |
| Excipient | 5 ml |

| Formulation No. 6 | |
|---|---|
| Bisphosphonate | 25 mg |
| Anti-IL-6 | 700 mg |
| Excipient | 25 ml |

| Formulation No. 7 | |
|---|---|
| Bisphosphonate | 25 mg |
| Anti-Lymphotoxin | 700 mg |
| Excipient | 5-25 ml |

| Formulation No. 8 | |
|---|---|
| Bisphosphonate | 5 mg |
| Anti-TNF | 70 mg |
| Excipient | 5-25 ml |

| Formulation No. 9 | |
|---|---|
| Bisphosphonate | 5 mg |
| Anti-PGE | 70-700 mg |
| Excipient | 5-25 ml |

| Formulation No. 10 | |
|---|---|
| Bisphosphonate | 5 mg |
| Anti-1,25 dihydroxy vitamin D₃ | 70-700 mg |
| Excipient | 5-25 ml |

| Formulation No. 11 | |
|---|---|
| Pyrophosphate | 5-25 mg |
| Anti-PTHrp | 70-700 mg |
| Excipient | 5-25 ml |

| Formulation No. 12 | |
|---|---|
| Etidronate | 5-25 mg |
| Anti-PTHrp | 100 mg |
| Excipient | 10 ml |

| Formulation No. 13 | |
|---|---|
| Clodronate | 5 mg |
| Anti-PTHrp | 200 mg |
| Excipient | 5 ml |

| Formulation No. 14 | |
|---|---|
| Pamidronate | 20 mg |
| Anti-PTHrp | 500 mg |
| Excipient | 20 ml |

| Formulation No. 15 | |
|---|---|
| Tiludronate | 15 mg |
| Anti-PTHrp | 600 mg |
| Excipient | 15 ml |

| Formulation No. 16 | |
|---|---|
| Alendronate | 25 mg |
| Anti-PTHrp | 700 mg |
| Excipient | 25 ml |

| Formulation No. 17 | |
|---|---|
| Risedronate | 15 mg |
| Anti-PTHrp | 350 mg |
| Excipient | 15 ml |

| Formulation No. 18 | |
|---|---|
| Etidronate | 2.5 g |
| Anti-PTHrp | 70 mg |
| Excipient | 25 ml |

| Formulation No. 19 | |
|---|---|
| Etidronate | 2.0 g |
| Anti-PTHrp | 300 mg |
| Excipient | 25 ml |

| Formulation No. 20 | |
|---|---|
| Antibody (chimeric) | 70-700 mg |
| Excipient | 5-25 ml |

| Formulation No. 21 | |
|---|---|
| Anti-PTHrp (chimeric) | 70-700 mg |
| Excipient | 5-25 ml |

| Formulation No. 22 | |
|---|---|
| Anti-TGFα (chimeric) | 70 mg |
| Excipient | 5 ml |

| Formulation No. 23 | |
|---|---|
| Anti-IL-1α (chimeric) | 700 mg |
| Excipient | 25 ml |

| Formulation No. 24 | |
|---|---|
| Anti-IL-1β (chimeric) | 700 mg |
| Excipient | 5 ml |

| Formulation No. 25 | |
|---|---|
| Anti-IL-6 (chimeric) | 700 mg |
| Excipient | 25 ml |

| Formulation No. 26 | |
|---|---|
| Anti-Lymphotoxin (chimeric) | 700 mg |
| Excipient | 5-25 ml |

| Formulation No. 27 | |
|---|---|
| Anti-TNF (chimeric) | 70 mg |
| Excipient | 5-25 ml |

| Formulation No. 28 | |
|---|---|
| Anti-PGE (chimeric) | 70-700 mg |
| Excipient | 5-25 ml |

| Formulation No. 29 | |
|---|---|
| Anti-1,25 dihydroxy vitamin D₃ (chimeric) | 70-700 mg |
| Excipient | 5-25 ml |

In the formulations of the invention each of the bisphosphonate components will act in a similar manner. Further, each of the antibody components would be expected to have some similarity of action. Thus, to provide formulations of the invention any given bisphosphonate or group of bisphosphonates can be combined with any given antibody or group of antibodies and obtain synergistic results in treatment. Particularly preferred formulations are injectable formulations comprised of an antibody selected from the group consisting of human antibody, a humanized antibody and a chimeric antibody and excipient - with or without a bisphosphonate.

### Cancer Cell Metastasis Model

The present inventors have developed a model of human breast cell cancer metastasis to bone that results in osteolysis. Nakai, M. *et al.* Cancer Res (1992) 52:5395-5399; Sasakai, A. *et al.* J Bone Min Res (1993) 8 (Supp 1) :No. 92. Tumor cells introduced into the left cardiac ventricle of nude mice can be shown to cause osteolytic lesions that can be seen by x-ray examination and that can be confirmed histologically. (The model is applicable to normal mice and nude mice). A375 melanoma cells and the human breast cancer cell line MDA231 have been used in this model. Using this model, it was demonstrated that bone metastasis could be prevented by a synthetic antagonist to laminin.

This model has also been used to demonstrate in vivo the metastatic nature of human neuroblastoma cells as described in an abstract by Yoneda, T. *et al.* J Bone Min Res (1994) 9 (Supp 1):S 293, B 225. Six weeks after inoculation with the neuroblastoma cells, x-rays showed lesions in the tibiae that were both osteolytic and osteoblastic, but there was no hypercalcemia or cachexia. Thus, cancer metastasis of the bone is clearly a complex situation which can generate both osteoblasts and osteoclasts and is not always associated with systemic disorders such as hypercalcemia or cachexia. Histologic examination showed that neuroblastoma cells occupied the bone marrow cavity, and there were numerous bone resorbing osteoclasts (which have not been demonstrated to exhibit PTH/PTH-rp receptors) on endosteal bone surfaces. Culture supernatants of the tumor cells stimulated bone resorption in organ cultures of fetal rat long bones and increased proliferation of osteoblastic osteosarcoma cells, which promote bone formation. The culture media contained significant levels of PTH-rp and high levels of IL-6. Osteoclasts have IL-6 receptors.

The number of osteolytic lesions is increased by enhancing PTH-rp expression of the MDA231 cells used in the model, but not diminished by MDA231 cells transfected with an antisense PTH-rp construct. Overexpressing clones were obtained by transfecting MDA231 cells with cDNA encoding human prepro PTH-rp; clones having diminished production of this peptide were obtained by transfecting the cell line with an antisense construct. A clone showing elevated PTH-rp expression (100 pM/24 hours) produced 16.3 ± 3.8 lesions radiographically at 3 weeks as compared with the antisense construct which secreted less than 0.3 pM/24 hours which produced only 7.6 ± .22 lesions. The number of lesions in mice administered unmodified MDA231 cells which produce 1.6-8.4 pM/24 hours PTH-rp is similar to the number of lesions in mice administered the MDA231 cells modified to contain the antisense construct, which produces less PTH-rp. No plasma concentrations of PTH-rp were detectable in any of the mice used in these studies; hypercalcemia was minimal and present only in mice harboring the PTH-rp-enhanced MDA231 cells. The present inventors have also shown that PTH-rp concentrations in serum-free media conditioned by MDA231 cells were increased two-fold when the cells were cultured on an extracellular matrix produced by bone cells. These results were reported by Guise, T.A. *et al.* J Bone Min Res (1994) 9 (Supp 1):S 128, No. 30.

Using the above-described model we have found that antibodies specific for PTH-rp are affective in inhibiting metastasis and ameliorating the effects of malignant cells localized in bone. This provides an effective pharmacological approach to treatment.

### Hypercalcemia Distinct From Osteolytic Metastasis

The above-described animal model is effective in demonstrating the distinction between humoral hypercalcemia of malignancy (HHM) and the metastasis/osteolysis (which may or may not result in hypercalcemia) that is the target of the anti-PTH-rp treatment described herein and the relevance of PTH-rp to such metastasis/osteolysis is clearly demonstrated in the murine model system described by the present inventors in Nakai *et al.* (*supra*). Two tumor cell lines were used in this model: RWGT-2 which is a human squamous cell carcinoma of the lung that produces large amounts of PTH-rp in culture (251 ± 8 pM) and MDA231, the human breast adenocarcinoma described above that produces low amounts in vitro (3.1 ± .3 pM). The cultured tumor cells were inoculated by intramuscular (10⁷ cells) or intracardiac injection (10⁵ cells) into athymic nude mice. The levels of calcium ion in blood and of PTH-rp in plasma were measured, along with obtaining skeletal radiographs and bone histology.

Mice inoculated intramuscularly with either cell line developed local tumors in three weeks without metastases. Mice bearing RWGT-2 developed hypercalcemia (2.44 ± .16 mM calcium) with increased PTH-rp (11.8 ± 2.2 pM). Mice bearing MDA231 remained normal calcemic (1.22 ± .03 mM) and PTH-rp was not increased in the plasma (0.69 ± .17 pM). Osteoclastic bone resorption occurred in the RWGT-2-bearing mice, but not in MDA231-bearing mice.

On the other hand, mice inoculated in the left ventricle with either cell line developed osteolytic lesions in 4 weeks. MDA231-bearing mice remained normal calcemic (1.22 ± .03 mM) as did mice bearing RWGT-2 (1.45 ± .19 mM). PTH-rp concentration in the plasma was not increased in the MDA231-bearing mice (0.88 ± .15 pM) and only slightly increased in RWGT-2-bearing mice (2.75 ± 2.68 pM). However, tumor was present adjacent to areas of increased osteoclastic bone resorption in both groups.

The results show that PTH-rp causes different syndromes depending on where it is produced; when produced by tumors at a distant site from bone at high levels (RWGT-2), plasma PTH-rp is increased and hypercalcemia occurs without metastatic bone disease. They state that when PTH-rp is produced locally by tumor cells in bone (MDA231) localized bone destruction, with or without hypercalcemia, occurs without necessarily an increase in circulating PTH-rp. On the other hand, the observed osteolysis could be caused in part by PTH-rp released by other cells in response to signals from the metastasized cancer; in addition, enhanced levels of PTH-rp may be produced by the metastasized cancer cells when in the environment of the bone matrix. It is nevertheless clear that the syndrome addressed by the method of the present invention is clearly different from HHM.

Subjects afflicted with conditions responsive to the method of the invention can readily be identified. In some cases, the nature of the disease itself is diagnostic. Endogenous osteolytic conditions such as osteopcrosis, osteomalacia, renal osteodystrophy and Paget's Disease are routinely diagnosed. The presence of osteolysis in general can be verified by ascertaining the presence of bone lesions using x-ray scans. Generally speaking, subjects who are diagnosed with cancers of bone origin or cancers which have metastasized to bone or which have the prognosis for metastasis to bone are suitable subjects. The prognosis may be made on the basis of the known nature of the tumor which can be ascertained by biopsy and classification, or may be determined independently by assaying a biopsied tumor for its capacity to produce PTH-rp at elevated levels in the presence of components associated with bone. As stated above, the present inventors have shown that the metastatic tumor MDA231 produces increased levels of PTH-rp when the cells are cultured on an extracellular matrix produced by bone cells.

Thus, by a variety of criteria, a subject exhibiting the particular syndrome treatable by the method of the invention may be ascertained.

The benefits of the treatment described herein are manifest. In addition to the direct effect of the anti-PTH-rp in reducing bone metastasis and the associated bone lesions, the secondary effects of the metastases and bone lesions are also addressed. Thus, subjects exhibiting metastasis and osteolysis may secondarily exhibit hypercalcemia due to the destruction of the bone; the fractured or weakened bones resulting from osteolysis may compress the neural networks in their immediate vicinity ultimately resulting in paralysis or paraplegia; the risk of pathologic fracture is obvious; cachexia can often result; and bone destruction is inevitably associated with pain. All of the foregoing directly or indirectly decrease survival prognosis. Mitigation of these negative effects results from the prevention and treatment of cancer metastasis to the bone environment by cancers originating from either bone or other tissues; from the prevention and treatment of cancer growth on the bone and from the resulting prevention and treatment of the bone lesions caused by the foregoing. The anti-PTH-rp which lies at the heart of the method of the present invention represents a successful focus of treatment despite the multiplicity of factors that have been suggested to be responsible for the symptomology described herein.

### Methods of Treatment - Specific Dosages

The methods of treatment in accordance with the present invention can be carried out, in general, by using the specific formulations described above and administering those formulations as described in the subsection "Formulations - General" also put forth above. In general, antibodies are formulated into injectable formulations such as a physiological saline solution which may include appropriate buffers and other minor components conventionally contained within injectable solutions. The precise amount of antibody given to the patient will vary depending on factors such as the age, sex, weight and condition of the patient as well as the patient's responsiveness to treatment. In general, antibodies are administered in injectable formulations in a dosage range of about 50 µg/kg - 50 mg/kg but more preferably 1 mg/kg - 10 mg/kg per dosage. The dosage is repeatedly administered to the patient over a period of time. In general, the dosage is administered on a daily basis but can be administered twice daily or can be administered less frequently such as every 3 to 10 days. Preferred administration would be on a daily basis over a period of 3 to 10 days. After determining patient responsiveness the treatment protocol might be repeated.

As indicated in the formulation section the antibody may be delivered simultaneously with a bisphosphonate in the same formulation. Alternatively, a separate injectable formulation of a bisphosphonate might be administered to the patient. Unlike the antibodies the amount of bisphosphonate administered will not generally, vary depending upon the age, sex, weight and condition of the patient. However, one should consider patient responsiveness. Typically, the bisphosphone will not be administered each and every time the antibody is administered. More typically, the bisphosphonate such as pamidronate disodium is administered in an amount of about 30 to 60 milligrams on a bi-weekly basis to a normal sized adult. Higher doses in the range of 60 to 90 milligrams can be administered monthly. Bisphosphonates are also, typically, administered intravenously. 60 milligrams of bisphosphonate solution may be infused into a patient over a period of 1 to 24 hours. After administration of a desired dose over a period of time the caretaker will generally wait some period before readministering more bisphosphonate in order to determine patient responsiveness. Typically, after an administration of 60 to 90 milligrams the caretaker will wait 3 to 6 months before a further administration of bisphosphonate. However, such might vary greatly depending on the particular needs of the patient.

The amount of bisphosphonated administered will, in general, vary greatly depending upon the particular bisphosphonate being administered. Those skilled in the art will be able to adjust dosage depending on the particular compound used. For example, pamidronate is approximately 100 times more potent than etidronate with respect to its ability to inhibit bone resorption. The above dosing information is based on the administration of pamidronate. Accordingly, substantially larger amounts of etidronate would need to be administered to obtain the same effect.

Patients with primary cancer tumors are often treated surgically. One of the dangers of surgical removal of a cancer tumor is that cancer cells will be spread throughout the patient's body. In order to aid in preventing this formulations of the present invention can be administered prior to, during or after surgery. More typically, the formulation would be administered prior to surgery. Thus, a formulation of antibody (e.g., anti-PTHrp) is administered by injection along with an administration of a bisphosphonate. Although it is atypical, bisphosphonates can also be administered orally. Further, it is pointed out that the present invention contemplates various forms of administration which would be pharmaceutically and physiologically acceptable.

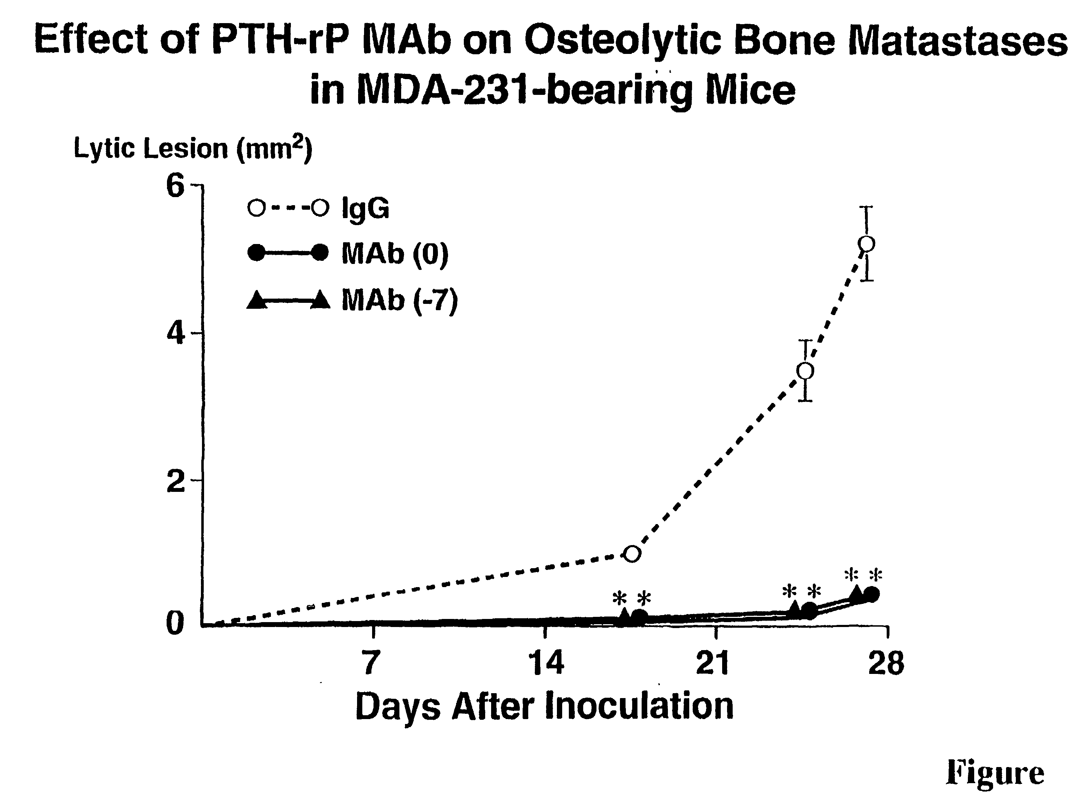

It should also be noted that the treatment protocols described herein can be carried out in connection with other diagnostic and treatment protocols. As an example, the present invention is particularly applicable with respect to patients which have been diagnosed, via X-rays, to have bone resorption due to cancer cell metastasis. When X-ray images of the patient show that the patient is suffering bone resorption, formulations of the invention including antibodies such as anti-PTHrp alone or in combination with bisphosphonates are administered. As shown in Figure 4 the use of anti-PTHrp has significant effects on decreasing bone resorption in patients with cancer.

### EXAMPLES

The following examples are put forth so as to provide those of ordinary skill in the art with a complete disclosure and description of how to make the formulations and carry out the methodology of the invention and are not intended to limit the scope of what the inventors regard as their invention. Efforts have been made herein to insure accuracy with respect to numbers used (e.g., amounts, numbers of cells, temperature, time, etc.) but some experimental errors and deviation should be accounted for. Unless indicated otherwise, parts are parts by weight, molecular weight is average molecular weight, units are conventional metric units, temperature is in degree centigrade and at approximately room temperature in the range of 18 to 25°C, and pressure is at or near atmospheric.

### Production of Antibodies Which Specifically Bind Human PTHrp

Antibodies specific for recombinant human PTHrp, or a fragment of naturally-occurring or genetically engineered human PTHrp, may be produced as follows. A polypeptide corresponding to all or part of PTHrp (recombinant or native) is produced by expression of all or a portion of the cDNA encoding human PTHrp in a host cell. Alternatively, human PTHrp, or peptide fragments thereof, may be produced by synthetic chemistry using standard techniques. Where desired, the peptides may be coupled to a carrier protein or other adjuvant to increase immunogenicity.

The peptide or peptide-carrier is mixed with Freund's adjuvant and injected into animals (e.g., mice, guinea pigs, rabbits) to produce polyclonal antibodies. Monoclonal antibodies which specifically bind recombinant human PTHrp protein, or fragments thereof, are generated using standard hybridoma technology (see, e.g., Kohler et al., 1975, *Nature* 256:495; Kohler et al., 1976, *Eur. J. Immunol.* 6:292; Kohler et al. 1976 *Eur. J. Immunol.* 6:511; Hammerling et al. in *Monoclonal Antibodies and T Cell Hybridomas,* (1981) Elsevier, NY; Ausubel et al., supra; Harlow and Lane, eds.; *Antibodies: A Laboratory Manual,* (1988) Cold Spring Harbor Laboratory, Cold Spring Harbor, NY). Once produced, antibodies are tested for specific binding to human PTHrp protein by Western blot, immunoprecipitation analysis, or other methods known in the art (see Ausubel et al., supra; Harlow and Lane, supra). Antibodies so produced are used in formulations and methods of treatment of the invention. Alternatively such antibodies may be labelled and injected and used to identify a site of bone resorption due to metastasis.

### Preparation A

### Effect of PTH-rp Monoclonal Antibody on Calcium Levels in an HHM Model

To confirm the effect of anti-PTH-rp in lowering calcium levels in HHM, mice were injected subcutaneously or intravascularly with either CHOK-1 cells or with bone-seeking squamous lung cancer cells designated RWGT-2 on day 0. For injection, the mice were under anesthesia with 0.05 mg/g pentobarbital. The cells (1 x 10⁵) were suspended in 0.1 ml PBS. On days 19 and 20 the mice received either 0.3 ml PBS or 0.3 ml containing 75µg antiPTH-rp (1-34) monoclonal antibodies (Sato *et al.* J Bone Min Res (1993) 8:849-860) subcutaneously.

Calcium levels in the plasma were measured at days 0, 7, 14, 17, 19 and 21. For the measurement, the mice were anesthetized and calcium ion was determined using a Ciba-Corning calcium pH analyzer (Model 634, Corning, Medfield, MA) as described by Yoneda, T. *et al.* J Clin Oncol (1991) 2:468-477.

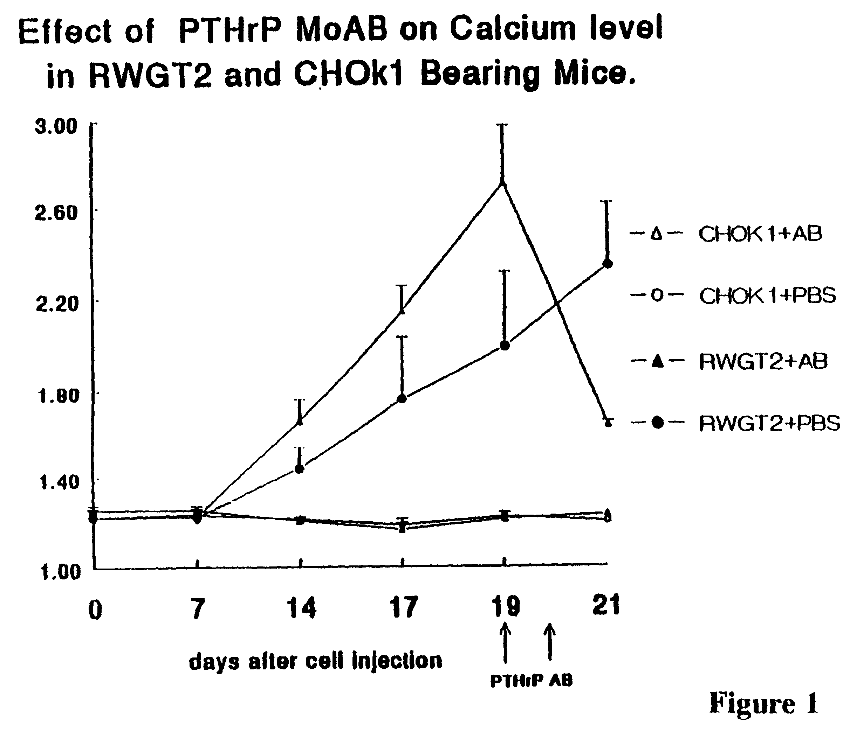

The results are shown in Figure 1. As shown, the control mice implanted with CHOK-l cells maintain normal calcium levels. Mice bearing RWGT-2 show continuously increasing calcium levels (solid circles) when only PBS is administered while administration of the anti-PTH-rp dramatically lowers the calcium levels in the blood of mice receiving antibody (solid triangles). Thus, in this HHM model, anti-PTH-rp controls hypercalcemia mediated by circulating PTH-rp.

### Example 1

### Effect of Anti-PTH-rp on Bone Resorption Stimulated by Various Agents

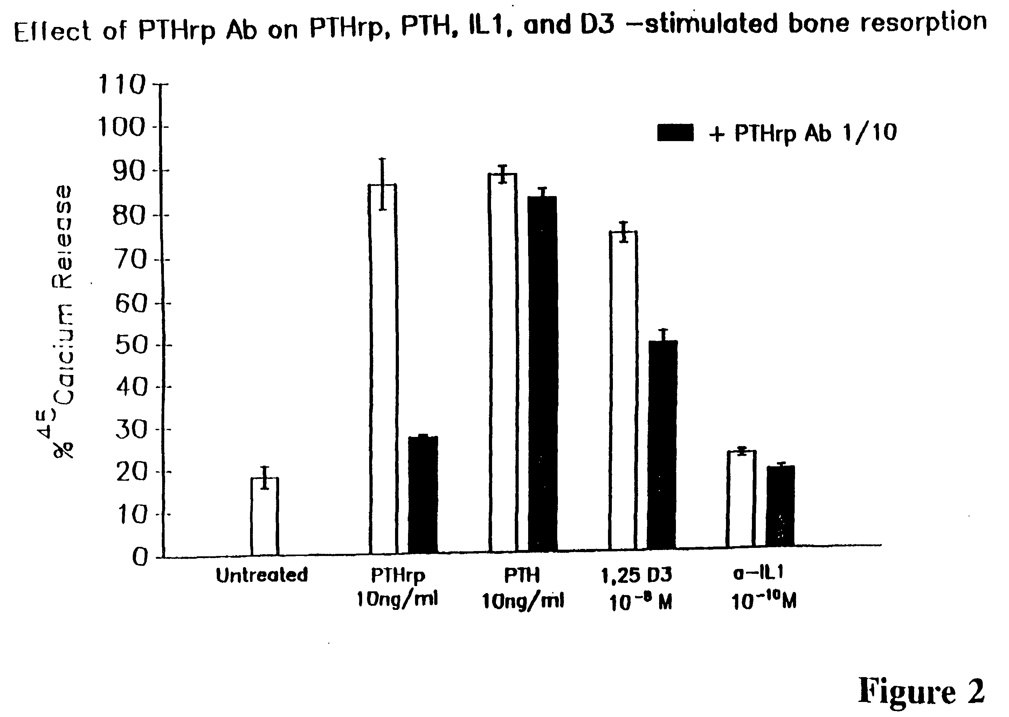

In this example, the percentage of labeled calcium (⁴⁵Ca) from fetal rat long bone was used as a measure of bone resorption. To label the bones, pregnant female rats at the 18th day of gestation were injected subcutaneously with 50 µCi of labeled calcium salt. The embryos were harvested the next day, at 19 days gestation, and long bones were obtained from the fetuses. The bones were cultured in standard media for 120 hours in the presence or absence of various putative bone resorption stimulation factors and in the presence and absence of the anti-PTH-rp (1-34) antibody used in Preparation A. The results are shown in Figure 2.

As shown, untreated bones released roughly 20% of the labeled calcium into the medium over the 120-hour time frame. The addition of 10 ng/ml of PTH-rp to the culture medium results in release of almost 90% of labeled calcium; however, addition of anti-PTH-rp antibodies diminishes this to roughly 30% Addition of 10 ng/ml PTH itself to the medium also results in almost 90% release of the calcium indicator of bone resorption but the antibody is sufficiently immunospecific to PTH-rp that there is essentially no effect by virtue of the presence of this antibody. The antibody also appeared to diminish the calcium release stimulated by 1,25 D-3 present at 10⁻⁸ M, however, these results could not be repeated. The addition of αIL-1 at 10⁻¹⁰ M had little effect on calcium release and the addition of the antibody was also without effect.

### Example 2

### Effect of Anti-PTH-rp on Long Bone Lesions

The osteolytic/metastatic model of Nakai, M. *et al.* Cancer Res (1992) 52:5395-5399 was used. MDA231 ± 4 cells were injected into the left ventricle with the mice under anesthesia with 0.05 mg/g pentobarbital on day 0. On days 14, 17 and 20, 0.3 ml anti-PTH-rp (1-34) (as per Preparation A described above) containing 75 µg protein or 0.3 ml PBS was injected subcutaneously. The area of bone lesions was estimated on radiographs; the mice were anesthetized deeply, laid down in prone position against the films (22 x 27 cm X-O Mart AR Kodak, Rochester, New York) and exposed with an x-ray at 35 kvp for 6 seconds using a Faxtron Radiographic Inspection Unit (Model 8050-020, Field Emission Corporation, Inc., McMinnville, OR). Films were developed using an RPX-O Mart Processor (Model M8B, Kodak). All radiographs were evaluated extensively by three different individuals. Metastatic foci, recognized as demarcated radiolucid lesions in bones, were enumerated. At the end of the experiment, the mice were sacrificed and bones were fixed in buffered 10% formalin and decalcified to confirm the presence of metastatic tumor and to examine the relationship between tumor and the related bones.

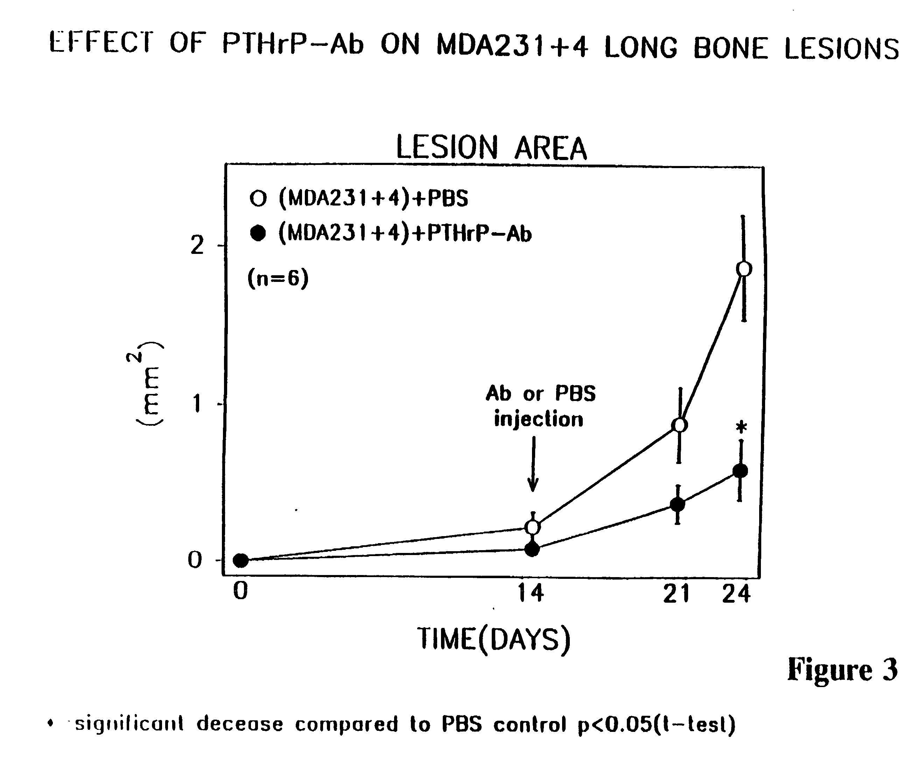

The results were tabulated as total lesion area in the bones of the fore- and hind-limbs and are shown as calculated for a single bone in Figure 3. As indicated, the total lesion area in a single bone, shown on the y-axis, is diminished when anti-PTH-rp is administered and is statistically significantly decreased at day 24.

### EXAMPLE 3

Four week-old female nude mice (Balb c Nu/Nu, Harlan Sprague-Dawley, Houston, TX) received the PTH-rPAb (MAb) 7 days prior to (-7) or at the same time (0) of inoculation of human breast cancer cells MDA-231 (1x10⁵ cells/animal) into the left cardiac ventricle. The anti-PTHrp (1-34) (as per Preparation A described above) antibodies or control IgG (75 µg/animal) were administered subcutaneously every 3 days for 28 days. Development of osteolytic lesions in extremities of these mice were monitored weekly by radiography using Faxitron^{R} (model 43855A, Faxitron X-ray Corp., Buffalo Grove, IL). Radiographs taken at day 17, 24 and 26 were examined for osteolytic lesions and area of the lesions in extremities of each mouse was quantified by computer-linked image analyzer. Data are expressed as area of lytic lesion (mm²) per mouse and mean ± S.E.M. of twelve nude mice for each treatment group. Statistical difference was analyzed by ANOVA followed by a paired test.

The results of following the above procedures are graphically shown in Figure 4. The control mice showed dramatic increases in osteolytic lesions (bone resorption) but the mice treated with anti-PTHrp show very small amounts of osteolytic lesions.

### EXAMPLE 4

### Mouse Marrow Cell Cultures

Mouse marrow cultures were carried out and the bone marrow cells were collected from femora and tibiae of C57BL mice (male, 4-6-wk old; Harlan Industries, Houston, TX), washed twice with serum-free alpha minimum essential medium (αMEM; Hazelton Biologic, Inc., Lenexa, KS), plated in 48-well plates (Falcon; Becton Dickinson; Labware, Lincoln Park, NJ) at a final density of 1.3 x 10⁶ cells/cm², and cultured in αMEM supplemented with 10% PCS (Hyclone Laboratories, Logan, UT), no antibiotics and 10 nM 1,25D₃ (Biomol Research Laboratory Inc., Plymouth Meeting, PA) for 6d at 37°C in a humidified atmosphere of 5% in air. The cells were fed every 2d, unless otherwise specified, with fresh αMEM containing 10% PCS and 10nM 1,25D₃.

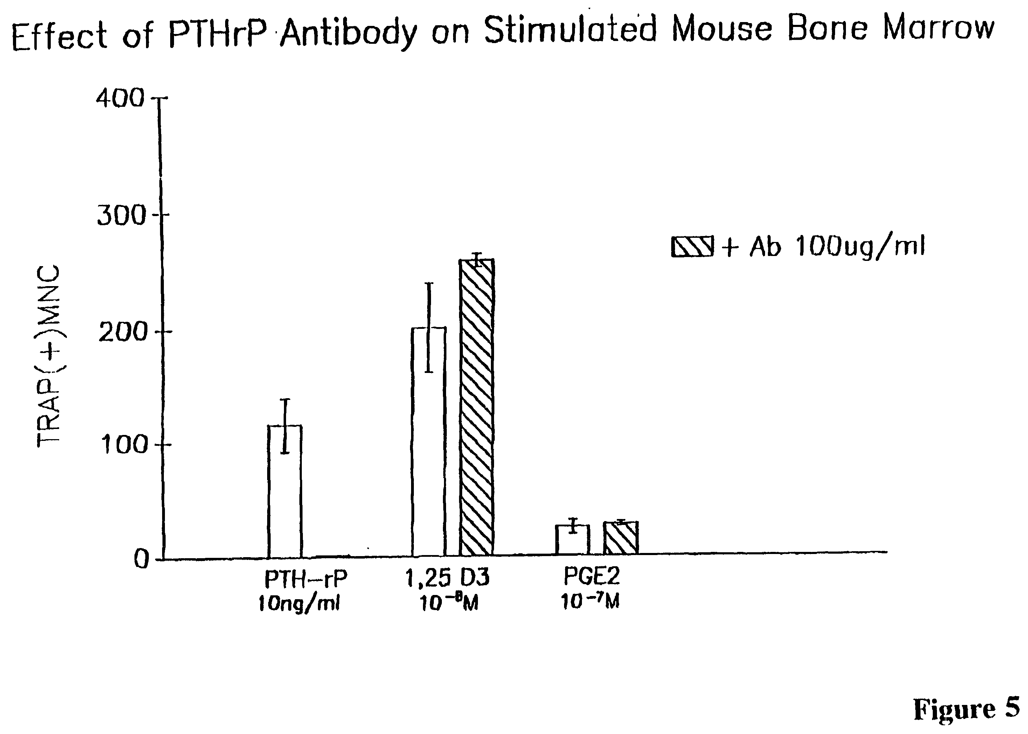

At the end of the culture, the cells were washed with PBS, fixed in 60% acetone in citrate buffer (pH 5.4) for 30 s, air dried, and stained for tartrate-resistant acid phosphatase (TRAP), which is a widely accepted cytochemical marker of murine osteoclasts (11), using a commercially available kit (Sigma). Stained cultures were examined under light microscopy at a magnification of 200. TRAP-positive (red-staining) multinucleated (three or more nuclei) cells (TRAP(+)MNC) in each well were counted by manually scanning across the entire well in a systematic fashion. Results obtained on the effects of anti-PTH-rp (1-34) antibody (as described in Preparation A above) of stimulated mouse bone marrow are shown in Figure 5.

### EXAMPLE 5

### Effects of Anti-PTHrp on Animal Model

Procedures were carried out in order to determine (1) the effect of cancer cell metastasized to the bone on PTHrp concentration; and (2) the effect of administering anti-PTHrp on metastasized cancer. Initially two groups of mice were provided. The first group (control group) was comprised of normal mice. The second group was comprised of the same type of normal mice as the first group which mice were subjected to the injection of cancer cells into the left ventricle as described above in the section entitled "Cancer Cell Metastasis Model." The group of mice having the cancer cells injected were treated and fed in the same manner as the control group other than the injection of the cancer cells. The injected cancer cells were allowed to metastasize to the bone.

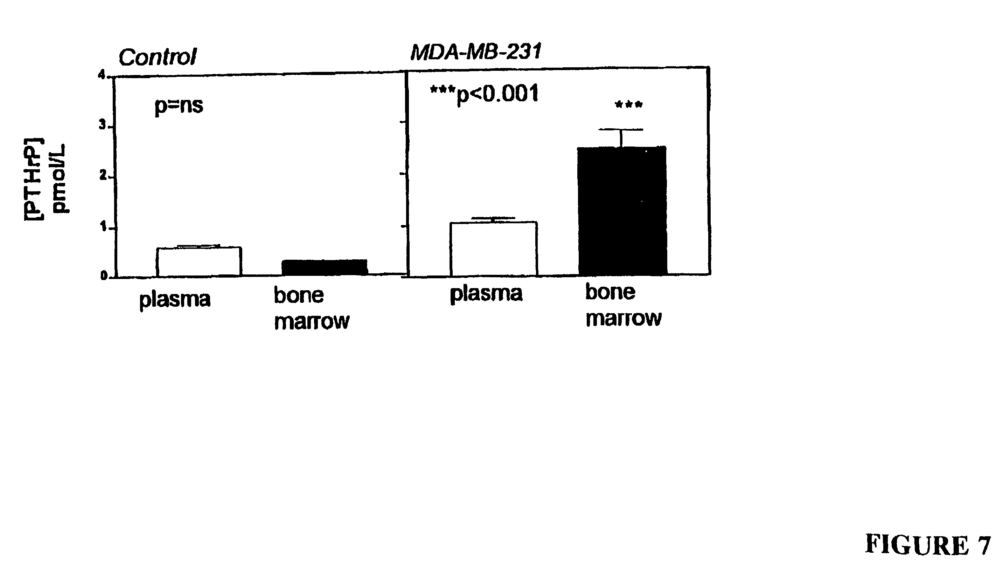

The peripheral blood PTHrp concentration as well as the bone marrow PTHrp concentration was measured in both the control group and the second group with the injected cancer cells. In the control group the PTHrp concentration was found to be less than 1 pico molar in both the peripheral blood and the bone marrow (see Figure 7). However, in the group having injected cancer cells the blood PTHrp concentration was also less than 1 pico molar whereas the local bone marrow PTHrp concentration was 3 pico molar (see Figure 7). Some animals showed even higher PTHrp concentration in the local bone marrow e.g., as high as 6 pico molar. The difference between the PTHrp concentration in the bone marrow between the control group and the group with the injected cancer cells was found to be statistically significant. Thus, the result showed that when cancer cells were allowed to metastasize to the bone the PTHrp concentration increased in a statistically significant fashion as compared with the PTHrp concentration in the bone marrow of the control group.

The second group of mice which had the injected cancer cells were then divided into two groups. The first group served as a new control group and the second group was treated in the same manner as the first group (new control group) except that the second group was treated with anti-PTHrp antibodies. In the control group i.e., the group with the cancer cells but not treated with anti-PTHrp antibody a histological examination of the cancer cells showed the cancer cells to be vital. However, in the group treated with anti-PTHrp antibody the histological examination of the cancer cells showed cell necrosis i.e:, localized cancer cell death. Thus, the results demonstrate, within the animal model, localized anti-cancer activity via the administration of anti-PTHrp antibodies.

The instant invention is shown and describe herein what is considered to be the most practical, and preferred embodiments.

## Claims

1. Use of an antibody or a fragment thereof immunoreactive and immunospecific for PTHrp or an antigenic fragment thereof for the manufacture of a medicament for preventing cancer metastasis to bone, inhibiting growth of cancer cells present in bone and/or for treating osteolysis.

2. Use according to claim 1 wherein the medicament further comprises an antibody which is immunoreactive and immunospecific for a compound which is TGFα, IL-1α, IL-1β, IL-6, Lymphotoxin, TNF, PGE, 1,25-dihydroxy vitamin D₃ or an antigenic fragment thereof.

3. Use according to claim 1 or 2 wherein the antibody, or antibodies, are chimeric and comprise a variable region derived from a non-human mammal and a constant region derived from a human.

4. Use according to any preceding claim wherein the antibody, or antibodies, are humanized and are derived from a transgenic mouse.

5. Use according to any of claims 1 to 4 wherein the antibody or antibodies are human.

6. Use according to any preceding claim wherein the medicament also comprises:
- a therapeutically effective amount of a biphosphonate or pyrophosphate of the general formula: 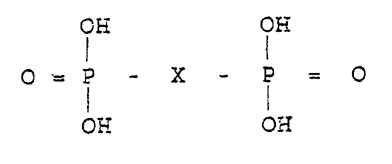
wherein X represents a linking moiety allowing for the interconnection of the phosphonate groups;
or pharmaceutically acceptable salt, hydrate or partial hydrate thereof.

7. Use according to claim 6 wherein X represents - O- or; 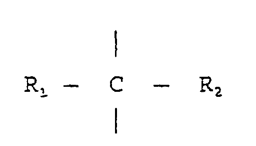 wherein each of R¹ and R² independently represent H, OH, C1, NH₂, S or thiophenyl where the phenyl ring may be substituted with a substituent which is C1, OH or NH₂; a C₁₋₁₂ alkyl group optionally substituted with O, S, NH₂, N or N-C₁₋₁₂ alkyl; a C₁₋₁₂ cyclic moiety any of whose carbon atoms are optionally substituted with C1, OH or NH₂; or a C₁₋₁₂ heterocyclic moiety any of whose carbon atoms are optionally substituted with C1, OH or NH₂ and where the hetoatom is O, N or S.

8. Use according to claim 6 wherein x represents - O-,

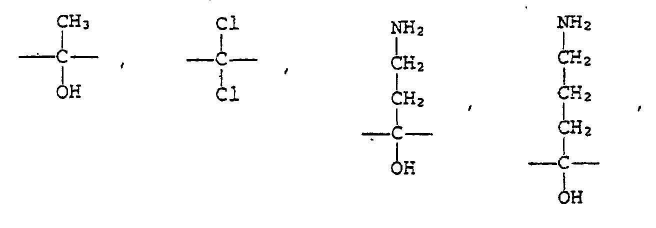

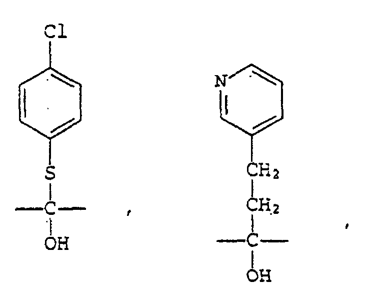

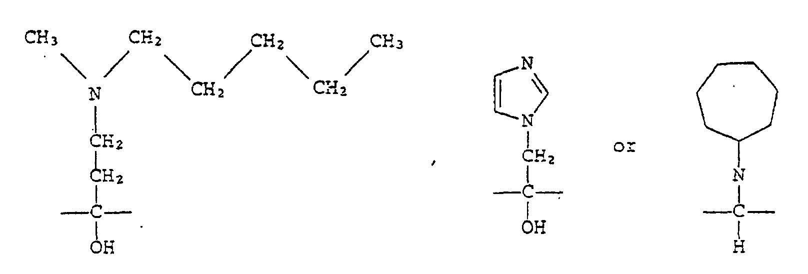

9. Use according to any preceding claim wherein the cancer cells have metastasised from a primary site other than bone.

10. Use according to claim 9 wherein the primary site cancer is of the breast, lung, kidney, head/neck, prostate, pancreas, skin, thyroid, testis, liver, urothelium, endometrium, cervix, oesophagus, blood, nervous system or lymphoid tissues.

11. Use according to any one of claims 1 to 8 wherein the cancer cells are of bone origin.

12. Use according to any of the preceding claims wherein the medicament treats a condition resulting from local PTHrp production and/or treats a secondary symptom.

13. Use according to claim 12 wherein the secondary symptom is:
- where a subject is suffering from pain and the inhibition alleviates the pain;
- where a subject is at risk for neural compression and the inhibition avoids or alleviates the compression;
- where a subject is hypercalcemic and the inhibition results in amelioration of the hypercalcemia;
- where the subject is at risk for mortality and the inhibition lengthens survival time;
- where a subject is at risk for pathologic fractures and the inhibition lessens the risk;
- where the subject has cachexia and the inhibition ameliorates the cachexia; or
- a combination of any of the foregoing.

14. Use according to claim 13, wherein the bone metastasis is a result of abnormal growth of a squamous cell, carcinoma cell, adenocarcinoma cell, melanoma cell, osteosarcoma cell, neuroblastoma cell or hematologic cancer cell.

15. Use according to any of the preceding claims wherein the medicament is for treating a patient with a primary cancer and the treatment comprises:
- surgically removing the primary cancer tumor; and
- administering to the patient prior to surgery, during surgery or after surgery an antibody immunoreactive and immunospecific for PTHrp or an antigenic fragment thereof.

16. Use according to any preceding claim wherein the treatment comprises:
- obtaining an X-ray image of a patient's bone;
- analysing the image to determine a site of bone resorption due to cancer cell metastasis; and
- administering the medicament to the patient.

## Patentansprüche

1. Verwendung eines gegenüber PTHrp oder einem antigenen Fragment davon immunreaktiven und immunspezifischen Antikörpers oder eines Fragments davon zur Herstellung eines Medikaments zur Verhinderung von Knochenkrebsmetastasen, Hemmung des Wachstums von in Knochen vorhandenen Krebszellen und/oder zur Behandlung von Osteolyse.

2. Verwendung nach Anspruch 1, wobei das Medikament weiter einen Antikörper umfaßt, der gegenüber einer Verbindung, bei der es sich um TGPα, IL-1α, IL-1β, IL-6, Lymphotoxin, TNF, PGE, 1,25-Dihydroxy-Vitamin D₃ oder ein antigenes Fragment davon handelt, immunreaktiv und immunspezifisch ist.

3. Verwendung nach Anspruch 1 oder 2, wobei der bzw. die Antikörper chimer ist bzw. sind und einen von einem nicht-menschlichen Säuger abgeleiteten variablen Bereich und einen von einem Menschen abgeleiteten konstanten Bereich umfaßt bzw. umfassen.

4. Verwendung nach einem vorhergehenden Anspruch, wobei der bzw. die Antikörper humanisiert ist bzw. sind und von einer transgenen Maus abgeleitet ist bzw. sind.

5. Verwendung nach einem der Ansprüche 1 bis 4, wobei der bzw. die Antikörper menschlich ist bzw. sind.

6. Verwendung nach einem vorhergehenden Anspruch, wobei das Medikament auch folgendes umfaßt:
- eine therapeutisch wirksame Menge eines Biphosphonats oder Pyrophosphats der allgemeinen Formel: 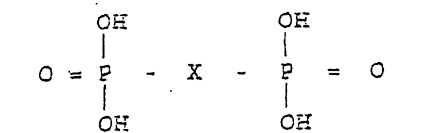 in der X eine Bindungseinheit, die eine Verknüpfung der Phosphonatgruppen untereinander ermöglicht;
oder ein pharmazeutisch verträgliches Salz, Hydrat oder Teilhydrat davon darstellt.

7. Verwendung nach Anspruch 6, wobei X -O- oder 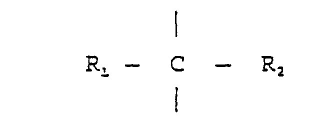 bedeutet, wobei jeder Rest R¹ und R² unabhängig H, OH, Cl, NH₂, S oder Thiophenyl bedeutet, wobei der Phenylring mit einem Substituenten substituiert sein kann, bei dem es sich um Cl, OH oder NH₂ handelt; einen C₁₋₁₂-Alkylrest, gegebenenfalls substituiert mit O, S, NH₂, N oder N-C₁₋₁₂-Alkyl, eine cyclische C₁₋₁₂-Einheit, bei der jedes Kohlenstoffatom gegebenenfalls mit Cl, OH oder NH₂ substituiert ist, oder eine heterocyclische C₁₋₁₂-Einheit, bei der jedes Kohlenstoffatom gegebenenfalls mit Cl, OH oder NH₂ substituiert ist und bei der das Heteroatom O, N oder S ist.

8. Verwendung nach Anspruch 6, wobei X -O- 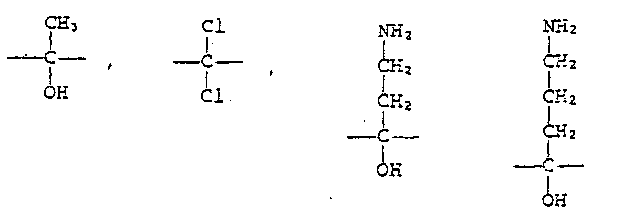 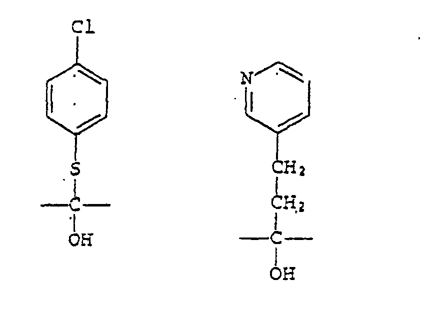 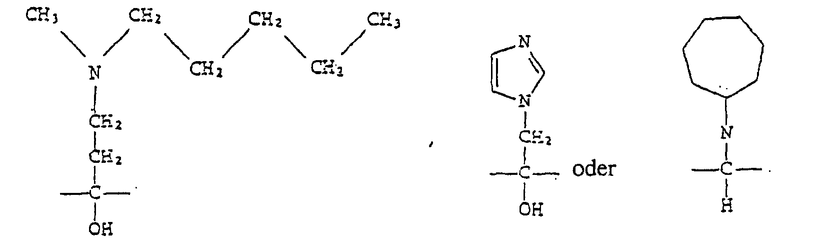 bedeutet.

9. Verwendung nach einem vorhergehenden Anspruch, wobei die Krebszellen ausgehend von einer anderen primären Auftrittsstelle als dem Knochen Metastasen bilden.

10. Verwendung nach Anspruch 9, wobei es sich bei dem Krebs an der primären Auftrittsstelle um Brust-, Lungen-, Nieren-, Kopf-/Nacken-, Prostata-, Bauchspeicheldrüsen-, Haut-, Schilddrüsen-, Hoden-, Leber-, Urothel-, Gebärmutter-, Gebärmutterhals-, Speiseröhren-, Blutkrebs, Krebs des Nervensystems oder Krebs des lymphatischen Gewebes handelt.

11. Verwendung nach einem der Ansprüche 1 bis 8, wobei die Krebszellen vom Knochen ausgehen.

12. Verwendung nach einem der vorhergehenden Ansprüche, wobei das Medikament einen aus lokaler PTHrp-Produktion resultierenden Zustand behandelt und/oder ein sekundäres Symptom behandelt.

13. Verwendung nach Anspruch 12, wobei das sekundäre Symptom folgende umfaßt:
- wenn ein Patient unter Schmerz leidet und die Hemmung den Schmerz lindert;
- wenn die Gefahr besteht, daß ein Patient eine neurale Kompression erleidet, und die Hemmung die Kompression verhindert oder lindert;
- wenn ein Patient hypercalcämisch ist und die Hemmung eine Linderung der Hypercalcämie zur Folge hat;
- wenn die Gefahr besteht, daß ein Patient stirbt, und die Hemmung die Überlebenszeit verlängert;
- wenn die Gefahr besteht, daß ein Patient pathologische Brüche erleidet, und die Hemmung das Risiko verringert;
- wenn der Patient unter Kachexie leidet und die Hemmung die Kachexie lindert; oder
- eine Kombination der Vorstehenden.

14. Verwendung nach Anspruch 13, wobei die Knochenmetastase, eine Folge des anormalen Wachstums einer Plattenepithelzelle, Karzinomzelle, Adenokarzinomzelle, Melanomzelle, Osteosarkomzelle, Neuroblastomzelle oder einer hamatoltische Krebszelle ist.

15. Verwendung nach einem der vorhergehenden Ansprüche, wobei das Medikament zur Behandlung eines Patienten mit einem Primärkrebs bestimmt ist und die Behandlung folgendes umfaßt:
- operative Entfernung des primären Krebstumors; und
- Verabreichung eines gegenüber PTHrp oder einem antigenen Fragment davon immunreaktiven und immunspezifischen Antikörpers an den Patienten vor der Operation, während der Operation oder nach der Operation.

16. Verwendung nach einem vorhergehenden Anspruch, wobei die Behandlung folgendes umfaßt:
- Erhalten einer Röntgenaufnahme des Knochens eines Patienten;
- Analysieren der Aufnahme, um eine Stelle von auf Krebszellenmetastase beruhender Knochenresorption zu bestimmen; und
- Verabreichen des Medikaments an den Patienten.

## Revendications

1. Utilisation d'un anticorps ou d'un fragment de celui-ci immunoréactif et immunospécifique à PTHrp ou un fragment antigénique de celle-ci pour la fabrication d'un médicament destiné à prévenir une métastase cancéreuse dans l'os, à inhiber la croissance de cellules cancéreuse présentes dans l'os et/ou à traiter une ostéolyse.

2. Utilisation selon la revendication 1, dans laquelle le médicament comporte de plus un anticorps qui est immunoréactif et immunospécifique à un composé qui est TGFα, IL-1α, IL-1β, IL-6, Lymphotoxine, TNF, PGE, 1,25-dihydroxy-vitamine D₃ ou un fragment antigénique de ceux-ci.

3. Utilisation selon la revendication 1 ou 2, dans laquelle l'anticorps ou les anticorps sont chimères et comportent une région variable dérivée d'un mammifère non-humain et une région constante dérivée d'un humain.

4. Utilisation selon l'une quelconque des revendications précédentes, dans laquelle l'anticorps ou les anticorps sont humanisés et sont dérivés d'une souris transgénique.

5. Utilisation selon l'une quelconque des revendications 1 à 4, dans laquelle l'anticorps ou les anticorps sont humains.

6. Utilisation selon l'une quelconque des revendications précédentes, dans laquelle le médicament comporte également :
- une quantité thérapeutiquement efficace d'un biphosphonate ou pyrophosphate ayant la formule générale suivante : 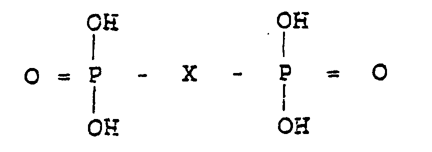
où X représente un fragment de liaison permettant l'interconnexion des groupes phosphonate,
ou un sel pharmaceutiquement acceptable, un hydrate ou un hydrate partiel de celui-ci.

7. Utilisation selon la revendication 6, où X représente -O- ou, 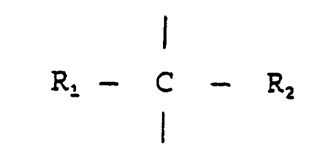 où R¹ et R² représentent chacun indépendamment H, OH, Cl, NH₂, S ou du thiophényle où l'anneau phénylique peut être substitué par un substituant qui est Cl, OH ou NH₂, un groupe alkyle C₁₋₁₂ substitué de manière facultative par O, S, NH₂, N ou un alkyle N-C₁₋₁₂, un fragment cyclique C₁₋₁₂ dont l'un quelconque des atomes de carbone est substitué de manière facultative par Cl, OH ou NH₂, ou un fragment hétérocyclique C₁₋₁₂ dont l'un quelconque des atomes de carbone est substitué de manière facultative par Cl, OH, ou NH₂ et où l'hétéroatome est O, N ou S.

8. Utilisation selon la revendication 6, où x représente -O-,

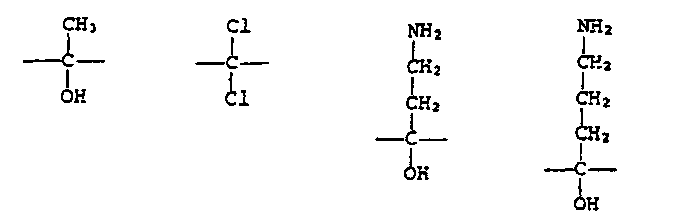

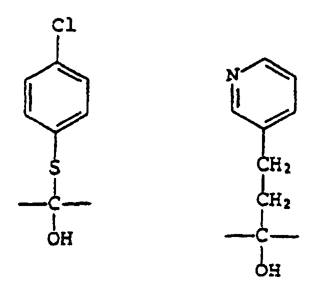

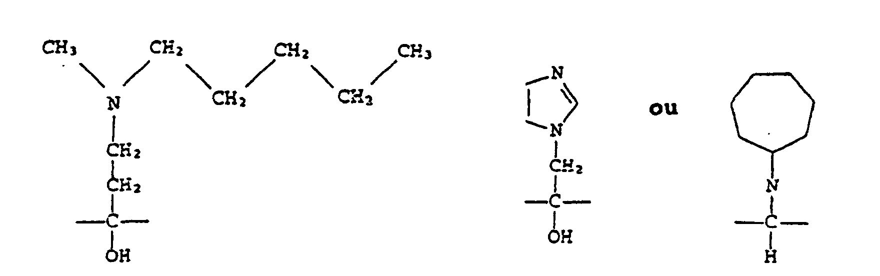

9. Utilisation selon l'une quelconque des revendications précédentes, dans laquelle les cellules cancéreuses ont été métastasées depuis un site primaire autre que l'os.

10. Utilisation selon la revendication 9, dans laquelle le cancer du site primaire est du sein, du poumon, du rein, de la tête/cou, de la prostate, du pancréas, de la peau, de la thyroïde, du testicule, du foie, de l'urothélium, de l'endomètre, du col de l'utérus, de l'oesophage, du sang, du système nerveux ou des tissus lymphoïdes.

11. Utilisation selon l'une quelconque des revendications 1 à 8, dans laquelle les cellules cancéreuses sont d'origine osseuse.

12. Utilisation selon l'une quelconque des revendications précédentes, dans laquelle le médicament traite une condition résultant d'une production de PTHrp locale et/ou traite un symptôme secondaire.

13. Utilisation selon la revendication 12, dans laquelle le symptôme secondaire est :
- lorsqu'un sujet souffre d'une douleur et l'inhibition soulage la douleur,
- lorsqu'un sujet présente un risque d'une compression neurale et l'inhibition évite ou soulage la compression,
- lorsqu'un sujet est hypercalcémique et l'inhibition a pour résultat une amélioration de l'hypercalcémie,
- lorsque le sujet présente un risque de mortalité et l'inhibition rallonge le temps de survie,
- lorsqu'un sujet présente un risque de fractures pathologiques et l'inhibition diminue le risque,
- lorsque le sujet a une cachexie et l'inhibition améliore la cachexie, ou
- une combinaison de l'un quelconque des symptômes précédents.

14. Utilisation selon la revendication 13, dans laquelle la métastase osseuse est un résultat d'une croissance anormale d'une cellule squameuse, d'une cellule de carcinome, d'une cellule d'adénocarcinome, d'une cellule de mélanome, d'une cellule d'ostéosarcome, d'une cellule de neuroblastome ou d'une cellule cancéreuse hématologique.

15. Utilisation selon l'une quelconque des revendications précédentes, dans laquelle le médicament est destiné à traiter un patient atteint d'un cancer primaire et le traitement comporte les étapes consistant à :
- éliminer de manière chirurgicale la tumeur cancéreuse primaire, et
- administrer au patient avant la chirurgie, pendant la chirurgie ou après la chirurgie, un anticorps immunoréactif et immunospécifique à PTHrp ou un fragment antigénique de celle-ci.

16. Utilisation selon l'une quelconque des revendications précédentes, dans laquelle le traitement comporte les étapes consistant à :
- obtenir une image aux rayons X d'un os du patient,
- analyser l'image pour déterminer un site de résorption osseuse due à une métastase de cellules cancéreuses, et
- administrer le médicament au patient.
